(19) Europäisches Patentamt
European Patent Office
Office européen des brevets

(11) EP 3 337 570 B1

(12) # EUROPÄISCHE PATENTSCHRIFT

(45) Veröffentlichungstag und Bekanntmachung des Hinweises auf die Patenterteilung:
**19.02.2020 Patentblatt 2020/08**

(21) Anmeldenummer: **16747759.5**

(22) Anmeldetag: **27.07.2016**

(51) Int Cl.:
***A61Q 5/12*** (2006.01) ***A61Q 5/02*** (2006.01)
***A61K 8/39*** (2006.01)

(86) Internationale Anmeldenummer:
**PCT/EP2016/067863**

(87) Internationale Veröffentlichungsnummer:
**WO 2017/029085 (23.02.2017 Gazette 2017/08)**

(54) **KONDITIONIERENDES SHAMPOO MIT ESTERGEMISCHEN VON PFLANZENÖLEN**
CONDITIONING SHAMPOO WITH ESTER-MIX OF PLANT OILS
SHAMPOOING DE CONDITIONNEMENT AVEC MÉLANGE D'ESTER D'HUILES VÉGÉTALES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2015 DE 102015215860**

(43) Veröffentlichungstag der Anmeldung:
**27.06.2018 Patentblatt 2018/26**

(73) Patentinhaber: **Henkel AG & Co. KGaA**
**40589 Düsseldorf (DE)**

(72) Erfinder:
- **FUHR, Denise**
  **22869 Schenefeld (DE)**
- **HENTRICH, Dirk**
  **22457 Hamburg (DE)**

(56) Entgegenhaltungen:
**EP-A1- 2 532 689** **EP-A2- 1 518 537**
**WO-A1-2005/030163** **WO-A1-2015/086006**

EP 3 337 570 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung liegt auf dem Gebiet der Kosmetik und betrifft konditionierende Haarreinigungsmittel, die ein Gemisch aus den Mono-, Di- und Triglyceriden einer bestimmten Fettsäuremischung und Glycerin sowie zusätzlich ein Gemisch aus den Mono- und Diestern dieser Fettsäuremischung und einem Polyethylenglycol enthalten. Die Haarreinigungsmittel sind weiterhin dadurch gekennzeichnet, dass sie weitere Pflanzenöle und Silikonöle nur in sehr kleinen Mengen enthalten und bevorzugt auf deren Einsatz verzichten.

Mithilfe kosmetischer Reinigungsmittel (Haarshampoos) können menschliche Haare und die Kopfhaut gereinigt und von Talg, Stylingmittelrückständen sowie andere Verschmutzungen befreit werden. Bedingt durch die in kosmetischen Haarreinigungsmitteln üblicherweise enthaltenen (meist anionischen) Tenside geht die Haarreinigung stets mit einer Entfernung von Lipiden und Proteinen aus den Haaren oder der Kopfhaut einher, wodurch insbesondere bei häufiger Reinigung eine Schädigung der Haarstruktur und/oder ein Austrocknen der Kopfhaut auftreten kann. Schädigungen der Haarstruktur oder der Haarfasern, insbesondere Spliss und/oder Haarbruch, können zudem durch Umwelteinflüsse (wie beispielsweise intensive Sonneneinstrahlung), mechanische Belastungen (wie beispielsweise Kämmen unter Föhnhitze) sowie durch chemische Einflüsse (wie beispielsweise Färben, Verformen oder Glätten der Haare) begünstigt werden.

Zur Verhinderung und/oder Minimierung von Haarschädigungen wurden Haarshampoos in der Vergangenheit bereits verschiedene Pflegestoffe wie beispielsweise kationische Pflegepolymere, Öle und/oder Silikonverbindungen hinzugefügt.

Mit all diesen Pflegestoffen kann ein Pflegeeffekt bzw. eine Konditionierwirkung erzielt werden, die sich beispielsweise in einem verbessertem Griff oder einer verbesserten Kämmbarkeit der Haare äußert. Gleichzeitig hat der Einsatz der vorgenannten Pflegestoffe jedoch auch verschiedene Nachteile, die dem Konsumenten negativ auffallen und ihn von der wiederholten Anwendung des Shampoos abhalten können.

So wird durch den Einsatz von größeren Ölmengen in Shampoos zwar die Kämmbarkeit verbessert, gleichzeitig beschweren Öle jedoch auch die Haare und lassen diese fettig wirken. Insbesondere bei wiederholter Anwendung kann eine Kumulation dieses Effektes auftreten, so dass die Haare bereits direkt nach dem Waschen und Trocknen ein ungepflegtes, unattraktives Erscheinungsbild aufweisen.

Der Einsatz von kationischen Tensiden und kationischen Polymeren kann zu einer elektrostatischen Aufladung und einem "Fliegen" der Haare führen. Auch dieser Effekt wird durch wiederholte Anwendung verstärkt.

Weiterhin sind Silikone für ihre Pflegewirkung bekannt. So betrifft beispielsweise WO 93/08787 A1 die Herstellung von Haarpflegeshampoos, die anionische Tenside, kationische Polymere, ein Silikon sowie ein weiteres Öl enthalten. Die Anwendung solcher Shampoos verleiht den Haaren mehr Glanz, eine verbesserte Kämmbarkeit und einen weicheren Griff. Die regelmäßige Verwendung von Pflegeshampoos dieses Typs kann jedoch zu der zuvor beschriebenen "Überpflegung" (so genannter Builtup-Effekt) der Haare führen. Darunter ist vor allem neben dem vorgenannten schmierigen Haargefühl auch ein geringes Haarvolumen und/oder ein strähniges, ungepflegtes Aussehen der Haare zu verstehen. Buit-up-Effekte treten besonders häufig auf feinen und/oder geschädigten Haaren auf und insbesondere nach der vielfachen Anwendung von Pflegeshampoos, die Silikone, Öle und kationische Polysaccharid-Polymere enthalten.

Dem Verbraucher sind die negativen Effekte dieser Pflegestoffe wohlbekannt, und neben Ölen haben insbesondere die Silikone bei manchem Verbraucher ein schlechtes Image. Viele Verbraucher sind daher auf der Suche nach Shampoos, die frei von Silikonen und/oder Ölen sind. Anhand der chemischen Bezeichnung bzw. der INCI ist es für den chemisch nicht vorgebildeten Verbraucher jedoch schwierig, die Inhaltsstoffe des Shampoos zu identifizieren, so dass er sich eine einfache und schnelle Möglichkeit wünscht, aus dem vorhandenen Angebot ein silikon- bzw. öl-freies Shampoo auswählen zu können.

Lipophile, pflegende Öle und Silikone müssen durch den Einsatz von Emulgatoren in der Formulierung stablisiert werden, so dass derartige Shampoos in der Regel in Form einer milchig trüben, nicht transparenten Emulsion vorliegen. Liegt das Pflege-Shampoo in transparenter Form, beispielsweise in Form eines Gels, vor, so ist es auch für den Verbraucher einfach zu erkennen, dass dieses Pflege-Shampoo keine Emulsion ist und demzufolge keine das Haar beschwerenden Fettstoffe enthält.

Transparente Shampoo Formulierungen, die keine lipophilen Öle und Silikone enthalten, sind prinzipiell auch aus dem Stand der Technik bekannt, jedoch handelt es sich bei diesen Shampoos lediglich um Reinigungs-Shampoos, nicht um Shampoos mit Konditionierwirkung.

Der vorliegenden Anmeldung lag daher die Aufgabe zugrunde, ein Haarreinigungsmittel mit hoher Pflegeleistung bereitzustellen, das die Haare nicht beschwert und nicht zu einer Überpflegung führt. Zur Vermeidung der Überpflegung soll die Pflegeleistung des Shampoos ohne den Einsatz von Silikonen und ohne große Ölmengen erzielt werden. Darüber hinaus soll das Shampoo dem Verbraucher eine einfache und schnelle Möglichkeit bieten, festzustellen, dass es keine Silikone/Öle enthält.

Es wurde nun überraschend gefunden, dass die zuvor genannten Aufgaben in hervorragendem Maße durch Haarreinigungsmittel gelöst werden, die neben den Mono-, Di- und Triglyceriden einer bestimmten Fettsäuremischung

und Glycerin zusätzlich ein Gemisch aus den Mono- und Diestern dieser Fettsäuremischung und einem Polyethylenglycol enthalten.

Diese Haarreinigungsmittel besitzen trotz ihres Verzichts auf Silikone und größere Ölmengen eine sehr hohe Pflegeleistung. Insbesondere können diese konditionierenden Shampoos den Griff und die Kämmbarkeit verbessern. Eine Überpflege bzw. ein negativer "Build-up" Effekt tritt auch nach wiederholter Anwendung dieser Shampoos nicht auf.

Schließlich können diese Shampoos als klare Formulierung hergestellt werden, d.h. es konnte ein transparentes konditionierendes Shampoo entwickelt werden, das dem Verbraucher bereits durch schnelle visuelle Beurteilung die Feststellung ermöglicht, dass lipophile, die Haare beschwerende Inhaltsstoffe nicht in diesem Shampoo enthalten sind.

Gegenstand der Erfindung ist daher ein konditionierendes Shampoo, enthaltend

(A) ein Gemisch aus den Mono-, Di- und Tri-Estern von einer Fettsäuremischung (F1) und Glycerin, und
(B) ein Gemisch aus den Mono- und Di-Estern einer Fettsäuremischung (F1) und einem Polyethylenglycol mit einer mittleren Molmasse von 200 bis 800 g/mol,
wobei

- es sich bei der Fettsäuremischung (F1) um ein Gemisch aus Fettsäuren handelt, das in seiner Zusammensetzung der Fettsäurezusammensetzung eines Pflanzenöls entspricht, und - bezogen auf das Gesamtgewicht des Shampoos -
- die Gesamtmenge aller im Shampoo enthaltenen Pflanzenöle, die von den Tri-Estern der Fettsäuremischung (F1) und Glycerin verschieden sind, bei einem Wert von maximal 0,25 Gew.-% liegt, und
- die Gesamtmenge aller im Shampoo enthaltenen Silikon-Verbindungen bei einem Wert von maximal 0,25 Gew.-% liegt, dadurch gekennzeichnet, dass es sich bei der Fettsäuremischung (F1) zwingend und ausschliesslich um ein Gemisch aus Fettsäuren handelt, das - bezogen auf das Gesamtgewicht der Fettsäuremischung (F1) - 4,0 - 8,0 Gew.-% Palmitinsäure, und 3,0 - 7,0 Gew.-% Stearinsäure, und 14,0 - 39,4 Gew.-% Ölsäure, und 60,0 - 88,0 Gew.-% Linolsäure umfasst.

Unter einem Shampoo wird im Sinne der Erfindung ein Haarreinigungsmittel verstanden, das in einem wässrigen oder wässrig/alkoholischen Träger mindestens ein Tensid, bevorzugt mindestens ein anionisches und/oder amphoteres und/oder zwitterionisches Tensid, enthält.

Unter einem konditionierenden Shampoo wird ein Haarreinigungsmittel verstanden, welches die Haare gleichzeitig reinigt und pflegt. Die konditionierende Wirkung bzw. Pflegewirkung äußert sich hierbei besonders bevorzugt in der Verbesserung des Griffgefühls und der Kämmbarkeit der Haare.

Tenside sind Substanzen, die die Oberlächenspannung einer Flüssigkeit oder die Grenzflächenspannung zwischen zwei Phasen herabsetzen. Tenside umfassen üblicherweise einen hydrophoben Kohlenwasserstoffrest (beispielsweise einem Kohlenwasserstoffrest mit 8 bis 30 Kohlenstoffatomen), und einem hydrophilen Molekülteil.

Anionische Tenside sind die am häufigsten in kosmetischen Reinigungsmitteln verwendeten Tenside, da sie außerordentlich schaumstark sind und eine hervorragende Reinigungsleistung aufweisen.

Zu den geeigneten anionischen Tensiden, die in den erfindungsgemäßen Shampoos eingesetzt werden können, zählen:

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel $R\text{-}(OCH_2\text{-}CH_2)_x\text{-}OSO_3^{-}X^{+}$, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

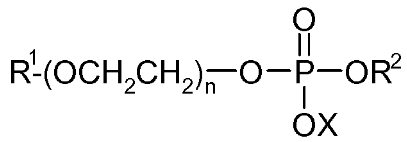

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 0 bis 10 und X für Wasserstoff, ein Alkali- oder Erdalkalimetall oder $NR^3R^4R^5R^6$, mit $R^3$ bis $R^6$ unabhängig voneinander stehend für einen $C_1$ bis $C_4$ -Kohlenwasserstoffrest, steht.

Bevorzugte anionische Tenside sind Ethercarbonsäuren der zuvor genannten Formel, Acylsarcoside mit 8 bis 24 C-Atomen in der Acylgruppe, Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel. Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten. Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Ganz besonders bevorzugte erfindungsgemäße Kosmetika sind dadurch gekennzeichnet, dass sie mindestens ein anionisches Tensid aus der Gruppe der Alkylsulfate und Alkylpolyglykolethersulfate der Formel $R\text{-}O\text{-}(CH_2\text{-}CH_2O)_n\text{-}O\text{-}SO_3X$ enthalten, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht, wobei anionische Tenside mit der INCI Bezeichnung Sodium Lauryl Sulfate besonders bevorzugt sind.

Bei dem kosmetischen Träger handelt es sich bevorzugt um einen geeigneten wässrigen, alkoholischen oder wässrig-alkoholischen Träger.

Der kosmetische Träger enthält bevorzugt mindestens 50 Gew.-%, mehr bevorzugt mindestens 60 Gew.-%, besonders bevorzugt mindestens 65 Gew.-% und insbesondere bevorzugt mindestens 70 Gew.-% Wasser. Weiterhin kann der kosmetische Träger 0,01 bis 30 Gew.-%, bevorzugt 0,05 bis 20 Gew.-% und insbesondere 0,1 bis 10 Gew.-% mindestens eines - von b) verschiedenen - Alkohols enthalten. Geeignete Alkohole sind beispielsweise Ethanol, Ethyldiglykol, 1-Propanol, 2-Propanol, Isopropanol, 1,2-Propylenglycol, 1,3-Propylenglycol, Glycerin, Diglycerin, Triglycerin, 1-Butanol, 2-Butanol, 1,2-Butandiol, 1,3-Butandiol, 1,4-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Besonders bevorzugt sind die wasserlöslichen Alkohole. Insbesondere bevorzugt sind Ethanol, 1,2-Propylenglycol, Glycerin, Benzylalkohol und/oder Phenoxyethanol sowie Mischungen dieser Alkohole.

Als ersten erfindungswesentlichen Bestandteil (A) enthalten die erfindungsgemäßen konditionierenden Shampoos ein Gemisch aus den Mono-, Di- und Tri-Estern von einer Fettsäuremischung (F1), wie sie in den Ansprüchen und vorstehend definiert wurde, und Glycerin. Unter der Fettsäuremischung (F1) wird eine Mischung aus zwei oder mehreren, linearen, verzweigten, gesättigten oder ungesättigten $C_8$-$C_{30}$-Alkansäuren oder $C_8$-$C_{30}$-Alkensäuren verstanden. Wenn eine oder mehrere Fettsäuren aus der Fettsäuremischung (F1) ungesättigt sind, so können die einfach oder mehrfach ungesättigt sein.

Bevorzugt handelt es sich bei der Fettsäuremischung (F1) um ein Gemisch aus mindestens zwei Fettsäuren, die aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure] ausgewählt werden.

Bei dem Gemisch (A) aus den aus den Mono-, Di- und Tri-Estern von der Fettsäuremischung (F1) und Glycerin handelt es sich um ein Gemisch aus den Verbindungen der Gruppen (A1), (A2) und (A3)

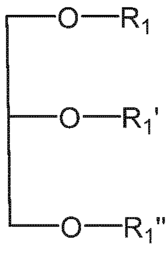 (A1) 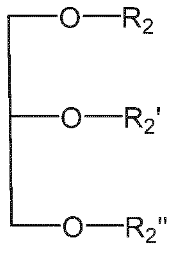 (A2) 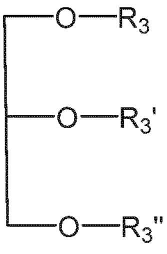 (A3)

wobei

- in jeder der Verbindungen aus der Gruppe (A1) die Reste R1, R1’ und R1" unabhängig voneinander für eine lineare, gesättigte oder einfach oder mehrfach ungesättigte $C_8$-$C_{30}$-Acylgruppe stehen, und
- in jeder der Verbindungen aus der Gruppe (A2) einer der Reste aus R2, R2’ und R2" für ein Wasserstoffatom steht und die anderen beiden Reste unabhängig voneinander für eine lineare, gesättigte oder einfach oder mehrfach ungesättigte $C_8$-$C_{30}$-Acylgruppe stehen, und
- in jeder der Verbindungen aus der Gruppe (A3) zwei der Reste aus R3, R3’ und R3" für ein Wasserstoffatom stehen und der dritte Rest für eine lineare, gesättigte oder einfach oder mehrfach ungesättigte $C_8$-$C_{30}$-Acylgruppe steht.

Jede C8-C30-Acylgruppe korrespondiert mit der entsprechenden Fettsäure. Umfasst die Fettsäuremischung (F1) beispielsweise Dodecansäure (Laurinsäure, $C_{12}H_{24}O_2$), so handelt es sich bei der korrespondierenden C8-C30-Acylgruppe um die Gruppe -C(O)-$C_{11}H_{23}$. Das offenbarte Shampoo enthält somit ein Gemisch aus einer oder mehreren Verbindungen der Gruppe (A1), zusätzlich ein Gemisch aus einer oder mehreren Verbindungen der Gruppe (A2) und zusätzlich ein Gemisch aus mindestens zwei Verbindungen der Gruppe (A3).

Die Summe aller im Mittel enthaltenen Verbindungen aus der Gruppe (A1) entspricht den Triestern der Fettsäuremischung (F1) und Glycerin, d.h. die Summe aller Reste R1, R1’ und R1" steht für die Acylreste, die Strukturbestandteil des Triesters aus der Fettsäuremischung (F1) und Glycerin sind. Die Summe aller im Mittel enthaltenen Verbindungen aus der Gruppe (A2) entspricht den Diestern der Fettsäuremischung (F1) und Glycerin, d.h. ein Drittel der Summe aller Reste R2, R2’ und R2" steht für Wasserstoff, und die verbleibenden 2/3 aller Reste R2, R2’ und R2" stehen für die Acylreste, die Strukturbestandteil des Diesters aus der Fettsäuremischung (F1) und Glycerin sind.

Die Summe aller im Mittel enthaltenen Verbindungen aus der Gruppe (A3) entspricht den Monoestern der Fettsäuremischung (F1) und Glycerin, d.h. zwei Drittel der Summe aller Reste R3, R3’ und R3" stehen für Wasserstoff, und das verbleibende 1/3 aller Reste R3, R3’ und R3" steht für die Acylreste, die Strukturbestandteil des Diesters aus der Fettsäuremischung (F1) und Glycerin sind. In jeder der Verbindungen aus der Gruppe (A1) können die Reste R1, R1’ und R1" jeweils unabhängig von den Resten der anderen Vertreter aus der Gruppe (A1) gewählt werden.

In jeder der Verbindungen aus der Gruppe (A2) können die Reste R2, R2’ und R2" jeweils unabhängig von den Resten der anderen Vertreter aus der Gruppe (A2) gewählt werden.

In jeder der Verbindungen aus der Gruppe (A3) können die Reste R3, R3’ und R3" jeweils unabhängig von den Resten der anderen Vertreter aus der Gruppe (A3) gewählt werden.

In einer besonders bevorzugten Ausführungsform ist das erfindungsgemäße konditionierende Shampoo dadurch gekennzeichnet, dass es sich bei dem Gemisch (A) aus den Mono-, Di- und Tri-Estern von der Fettsäuremischung (F1) und Glycerin um ein Gemisch aus den Verbindungen der Gruppen (A1), (A2) und (A3) handelt,

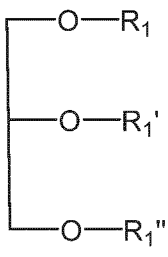 (A1) 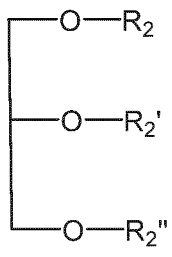 (A2) 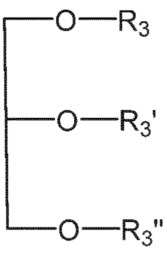 (A3)

wobei

- in jeder der Verbindungen aus der Gruppe (A1) die Reste R1, R1’ und R1" unabhängig voneinander für eine lineare, gesättigte oder einfach oder mehrfach ungesättigte $C_8$-$C_{30}$-Acylgruppe stehen, und

- in jeder der Verbindungen aus der Gruppe (A2) einer der Reste aus R2, R2' und R2" für ein Wasserstoffatom steht und die anderen beiden Reste unabhängig voneinander für eine lineare, gesättigte oder einfach oder mehrfach ungesättigte $C_8$-$C_{30}$-Acylgruppe stehen, und
- in jeder der Verbindungen aus der Gruppe (A3) zwei der Reste aus R3, R3' und R3" für ein Wasserstoffatom stehen und der dritte Rest für eine lineare, gesättigte oder einfach oder mehrfach ungesättigte $C_8$-$C_{30}$-Acylgruppe steht.

Bei der Fettsäuremischung (F1) handelt es sich um ein Gemisch aus mindestens zwei verschiedenen - bevorzugt mindestens drei verschiedenen - Fettsäuren.

Als Triester mit Glycerin bildet diese Fettsäuermischung die Verbindungen aus der Gruppe (A1) aus. Im Prinzip kann das konditionierende Shampoo daher nur eine Verbindung aus der Gruppe (A1) enthalten, bei welcher dann jedoch mindestens zwei der Reste aus der Gruppe R1, R1' und R1" strukturell verschieden sein müssen. Es ist jedoch bevorzugt, wenn das konditionierende Shampoo mindestens zwei strukturell verschiedene Verbindungen aus der Gruppe (A1) enthält.

Im Prinzip kann das konditionierende Shampoo auch nur eine Verbindung aus der Gruppe (A2) enthalten, bei welcher dann jedoch die beiden Reste aus der Gruppe R2, R2' und R2", die kein Wasserstoffatom darstellen, strukturell verschieden sein müssen. Es ist jedoch bevorzugt, wenn das konditionierende Shampoo mindestens zwei strukturell verschiedene Verbindungen aus der Gruppe (A2) enthält.

Weiterhin enthält das konditiorende Shampoo mindestens zwei strukturell verschiedene Verbindungen aus der Gruppe (A3).

Als zweiten erfindungswesentlichen Bestandteil (B) enthält das konditionierende Shampoo ein Gemisch aus den Mono- und Di-Estern einer Fettsäuremischung (F1) und einem Polyethylenglycol mit einer mittleren Molmasse von 200 bis 800 g/mol.

Die Fettsäuremischung (F1) des Bestandteils (B) entspricht der Fettsäuremischung (F1) des Bestandteils (A).

Würden also alle Ester des Bestandteils (A) vollständig gespalten und alle Ester des Bestandteils (B) vollständig gespalten, so hätten die aus der Esterspaltung der Bestandteile (A) und (B) resultierenden Fettsäuregemische beide dieselbe Zusammensetzung (F1).

Der Bestandteil (B) stellt ein Gemisch aus den Mono- und Di-Estern von der Fettsäuremischung (F1) und einem Polyethylenglycol mit einer mittleren Molmasse von 200 bis 800 g/mol dar.

Ein Polyethylenglycol mit einer mittleren Molmasse von 200 g/mol bis 800 g/mol ist ein Gemisch aus Verbindungen aus der Gruppe $H-(O-CH_2-CH_2)_n-OH$, wobei n beispielsweise für eine ganze Zahl von 1 bis 100 stehen kann, und wobei n bevorzugt für eine ganze Zahl von 1 bis 50, bevorzugt von 2 bis 40, weiter bevorzugt von von 3 bis 30 und besonders bevorzugt von 4 bis 20 steht. Im Mittel liegt die Molmasse diseses Gemisches im Bereich von 200 bis 800 g/mol.

Jedes der Polyethylenglycole aus dem eingesetzten Gemisch besitzt - abhängig von der Zahl n - ein bestimmtes Molgewicht. Das Gemisch der Polyethylenglycole ist durch seine mittlere Molmasse gekennzeichnet.

Die Molmasse (alternativ: das Molgewicht) ist definiert als Masse pro Stoffmenge (Einheit: g/mol). Die mittlere Molmasse (alternativ: das mittlere Molgewicht) des Gemisches der Polyethylenglycole ergibt sich aus der Gesamtmasse des Gemisches (d.h. der Summe der Massen aller einzelnen Polyethylenglycole) die zu der gesamten Stoffmenge des Gemisches in Relation gesetzt wird (d.h. der Gesamtanzahl aller im Gemisch vorhandenen Polyethylenglycole).

mittlere Molmasse = Gesamtmasse (g gesamt) / Gesamt-Stoffmenge (mol gesamt)

Bei Polymeren ist die mittlere Molmasse eine Kenngröße der Molmassenverteilung, wobei die Molmassen sämtlicher Kettenlängen gemittelt werden. Der erfindungsgemäß verwendete Begriffe mittlere Molmasse wird alternativ auch als Zahlenmittel der Molmasse bezeichnet. Die mittlere Molmassse (alternativ: das mittlere Molgewicht) des Gemisches aus Polyethylenglycolen kann beispielsweise durch Gelpermeationschromatographie (GPC) mit Polystyrol als internem Standard gemäß DIN 55672-3 bestimmt werden.

Bei dem Gemisch (B) aus den aus den Mono- und Di-Estern von der Fettsäuremischung (F1) und einem Polyethylenglycol mit einer mittleren Molmasse von 200 bis 800 g/mol handelt es sich um ein Gemisch aus den Verbindungen der Gruppen (B1) und (B2)

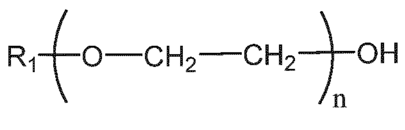 (B1) 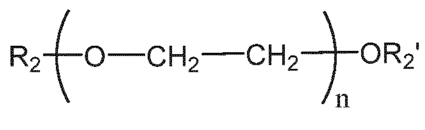 (B2)

wobei

- in jeder der Verbindungen aus der Gruppe (B1) der Rest R1 für eine lineare, gesättigte oder einfach oder mehrfach ungesättigte $C_8$-$C_{30}$-Acylgruppe steht, und
- in jeder der Verbindungen aus der Gruppe (B2) die Reste R2 und R2' unabhängig voneinander für eine lineare, gesättigte oder einfach oder mehrfach ungesättigte $C_8$-$C_{30}$-Acylgruppe stehen, und
- in jeder der Verbindungen aus den Gruppen (B1) und (B2) n unabhängig für eine ganze Zahl von 1 bis 50, bevorzugt von 2 bis 40, weiter bevorzugt von von 3 bis 30 und besonders bevorzugt von 4 bis 20 steht.

Hierbei muss wie zuvor beschrieben die Maßgabe erfüllt sein, dass das mittlere Molgewicht der eingesetzten Polyethylenglycole eine mittlere Molmasse von 200 bis 800 g/mol besitzt.

In jeder der Verbindungen aus der Gruppe (B1) können die Reste R1 und n jeweils unabhängig von den Resten R1 und n der anderen Vertreter aus der Gruppe (B1) gewählt werden.

In jeder der Verbindungen aus der Gruppe (B2) können die Reste R2, R2' und n ebenfalls jeweils unabhängig von den Resten der anderen Vertreter R, R2# und n aus der Gruppe (B2) gewählt werden.

Das hier offenbarte konditionierende Shampoo ist dadurch gekennzeichnet, dass es sich bei dem Gemisch (B) aus den Mono- und Di-Estern von der Fettsäuremischung (F1) und einem Polyethylenglycol mit einer mittleren Molmasse von 200 bis 800 g/mol um ein Gemisch aus den Verbindungen der Gruppen (B1) und (B2) handelt,

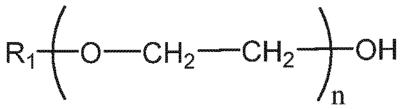
(B1)

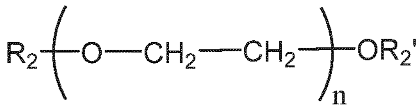
(B2)

wobei

- in jeder der Verbindungen aus der Gruppe (B1) der Rest R1 für eine lineare, gesättigte oder einfach oder mehrfach ungesättigte $C_8$-$C_{30}$-Acylgruppesteht, und
- in jeder der Verbindungen aus der Gruppe (B2) die Reste R2 und R2' unabhängig voneinander für eine lineare, gesättigte oder einfach oder mehrfach ungesättigte $C_8$-$C_{30}$-Acylgruppe stehen, und
- in jeder der Verbindungen aus den Gruppen (B1) und (B2) n unabhängig für eine ganze Zahl von 5 bis 12, bevorzugt von 6 bis 11, und besonders bevorzugt von 7 bis 10 steht.

Das hier offenbarte konditionierende Shampoo enthält ein Gemisch (A), bei dem es sich um ein Gemisch aus den Mono- und Di- und Tri-Estern von einer Fettsäuremischung (F1) und Glycerin handelt. Weiterhin enthält ein hier dergestalt offenbartes konditionierendes Shampoo auch ein Gemisch (B), bei dem es sich um ein Gemisch aus den Mono- und Di-Estern einer Fettsäuremischung (F1) und einem Polyethylenglycol mit einer mittleren Molmasse von 200 bis 800 g/mol handelt. Beiden Gemischen (A) und (B) liegt die Fettsäuremischung (F1) zugrunde.

Hierbei ist ein wesentliches Merkmal der Fettsäuremischung (F1), dass sie ein Gemisch aus Fettsäuren darstellt, das in seiner Zusammensetzung der Fettsäurezusammensetzung eines Pflanzenöls entspricht.

Bei Pflanzenölen handelt es sich um die Gemische von Fettsäuretriglyceriden, die beispielsweise durch Extraktion oder Pressung der Samen, Kerne oder des Fruchtfleisches einer Pflanze erhalten werden können. Pflanzenöle sind bei Raumtemperatur (22 °C) und Normaldruck (1 atm bzw. 1013 mbar) wachsartig bis flüssig, bevorzugt flüssig.

Werden diese Fettsäuretriglyceride - beispielsweise durch Hydrolyse - in ihre Bestanteile aufgespalten, so werden Glycerin und das für die jeweilige Pflanze charakteristische Fettsäuregemisch erhalten.

Shampoos mit besonders hoher Pflegewirkung konnten entwickelt werden, wenn die Gemische (A) und (B) auf einer Fettsäuremischung (F1) basieren, die in ihrer Zusammensetzung der Fettsäurezusammensetzung eines Öls aus der Gruppe aus Mandelöl, Aprikosenkernöl, Arganöl, Avocadoöl, Bassusöl (Orbignya Oleifera Kernöl), Paranussöl (Bertholletia Excelsa Kernöl), Bitterkirschen Kernöl, Traubenkernöl, Wassermelonenkernöl, Macadamianussöl (Macadamia Ternifolia Kernöl), Marulaöl (Sclerocarya Birrea Kernöl), Manketti-Öl (Schinzipphyton Rautenii Kernöl), Sonnenblumenöl, Olivenöl, Hagebuttenkernöl (Rosa Rubiginosa Kernöl) oder Distelkernöl entspricht.

In einer weiteren besonders bevorzugten Ausführungsform ist das erfindungsgemäße konditionierende Shampoo dadurch gekennzeichnet, dass es sich bei der Fettsäuremischung (F1) um ein Gemisch aus Fettsäuren handelt, das in seiner Zusammensetzung der Fettsäurezusammensetzung eines Öls aus der Gruppe aus Mandelöl, Aprikosenkernöl, Arganöl, Avocadoöl, Bassusöl (Orbignya Oleifera Kernöl), Paranussöl (Bertholletia Excelsa Kernöl), Bitterkirschen Kernöl, Traubenkernöl, Wassermelonenkernöl, Macadamianussöl (Macadamia Ternifolia Kernöl), Marulaöl (Sclerocarya Birrea Kernöl), Maneketti-Öl (Schinzipphyton Rautenii Kernöl), Sonnenblumenöl, Olivenöl, Hagebuttenkernöl (Rosa Rubiginosa Kernöl) oder Distelkernöl entspricht.

Unter dem Namen Mandelöl (*Prunus amygdalus dulcis (Sweet Almond) Oil*) versteht man das sowohl aus den süßen (lat. *dulcis*) als auch aus den bitteren (lat. *amarus*) Mandeln (beispielsweise durch Kaltpressung) gewonnene

Pflanzenöl. Das Fettsäuregemisch von Mandelöl enthält typischerweise max. 0,1 Gew.-% gesättigte Fettsäuren mit einer Kettenlänge von weniger als 16 C-Atomen, 4,0 - 9,0 Gew.-% Palmitinsäure, max. 0,8 Gew.-% Palmitoleinsäure, max. 0,2 Gew.-% Margarinsäure, max. 3,0 Gew.-% Stearinsäure, 62,0 - 86,0 Gew.-% Ölsäure, 20,0 - 30,0 Gew.-% Linolsäure, max. 0,4 Gew.-% Linolensäure, max. 0,2 Gew.-% Arachinsäure, max. 0,3 Gew.-% Eicosensäure, max. 0,2 Gew.-% Behensäure und max. 0,1 Gew.-% Erucasäure.

Aprikosenkernöl ist ein aus Aprikosenkernen gewonnenes Pflanzenöl. Das Fettsäuregemisch von Aprikosenkernöl enthält typischerweise 65,0 - 66,0 Gew.-% Ölsäure, 25,0 - 26,0 Gew.-% Linolsäure, 5,0 - 6,0 Gew.-% Palmitinsäure, 1,0 - 2,0 Stearinsäure und 0,1 - 1,0 Gew.-% Palmitoleinsäure enthält.

Das Arganöl (auch Arganienöl) wird in der Regel aus den Samen der gelben Beerenfrucht des Arganbaums (A. spinosa) durch Pressung gewonnen. Das Fettsäuregemisch von Agranöl enthält typischwerweise 44,0 - 46,0 Gew.-% Ölsäure, 33,0 - 35,0 Gew.-% Linolsäure, max. 0,5 Gew.-% Linolensäure, 14,0 - 18,0 Gew.-% Palmitinsäure, 5,0 - 6,0 Gew.-% Stearinsäure, max. 0,3 Gew.-% Arachidonsäure, max. 0,4 Gew.-% Gadolenäure.

Avocadoöl wird aus dem Fruchtfleisch der Avodaco gewonnen. Die Fettsäuren in Avocadoöl setzen sich in der Regel aus 47,0 bis 70,0 Gew.-% Ölsäure, 9,0 - 15,0 Gew.-% Linolsäure, 15,0 - 23,0 Gew-% Palmitinsäure und 5,0 - 13,0 Gew.-% Palmitoleinsäure zusammen.

Bassusöl (Orbignya Oleifera Kernel oil) ist das Öl der Samen von Orignya oleifera, auch Babassu Palme genannt. Ein tpisches Fettsäuregemisch von Babassuöl enthält üblicherweise ca. 39,0 - 41,0 Gew.-% Laurinsäure, ca. 15,0 Gew.-% Myristinsäure, ca. 16,0 Gew.-% Ölsäure, ca. 4,0 Gew.-% Linolsäure, ca. 4,0 Gew.-% Caprylsäure und ca. 4,0 Gew.-% Caprinsäure.

Das Paranussöl (Bertholletia Excelsa Kernöl) kann aus den getrockneten geschälten Samen, normalerweise durch Kaltpressung, gewonnen werden. Üblicherweise setzt sich das Fettsäuregemisch von Paranussöl aus den Fettsäuren 14,0 - 16,0 Gew.-% Palmitinsäure, 6,0 - 10,0 Gew.-% Stearinsäure, 29,0 - 48,0 Gew.-% Ölsäure und 30,0 - 47,0 Linolsäure zusammen Traubenkernöl, ist ein Öl, das aus den Kernen der Weintraubne gewonnen wird. Es kann durch Heißpressung oder durch Kaltpressung gewonnen werden. Das Fettsäuregemisch des Traubenkernöls setzt sich typischerweise aus 7,4 - 10,2 Gew.-% Palmitinsäure, 3,0 - 4,7 Gew.-% Stearinsäure, 16,1 - 21,6 Gew.-% Ölsäure und 63,3 - 71,4 Gew.-% Linolsäure zusammen. Wassermelonenkernöl wird synonym auch als Karingda Oil, Oontanga Oil oder Watermelon Seed Oil bezeichnet. Wassermelonenkernöl besitzt üblicherweise eine Fettsäurezusammensetzung von 11,0 - 19,0 Gew.-% Palmitinsäure, 13,0 - 17,0 Gew.-% Stearinsäure, 14,0 - 22,0 Gew.-% Ölsäure und 52,0 - 58,0 Gew.-% Linoslsäure.

Macadamia ist eine Pflanzengattung in der Familie der Silberbaumgewächse (Proteaceae). Sie ist vor allem durch die Frucht, die Macadamianuss bekannt. Aus der Macadamianuss kann das Öl beispielsweise durch Kaltpressung gewonnnen werden. Macadamianussöl (Macadamia Ternifolia Kernöl) besitzt üblicherweise eine Fettsäurezusammensetzung von 0,5 - 1,5 Ge.w-% Myristinsäure, 8,0 - 9,0 Gew.-% Palmitinsäure, 2,5 - 3,5 Gew.-% Stearinäsure, 55,0 - 58,0 Gew.-% Ölsäure, 20,0 - 22,0 Gew.-% Palmitoleinsäure und 1,5 - 2,5 Gew.-% Linolsäure zusammen.

Der Marula-Baum gehört zur Familie der *Anacardiaceae,* der Sumachgewächse und damit zur gleichen Familie wie Mango (Mangifera indica), Akajou-Baum (Anacardium occidentale, Cashew) und Pistazienbaum (Pistacia vera). Seine etwa pflaumengroßen, gelben Steinfrüchte enthalten ein aromatisches, süßsaures und weiß-durchscheinendes Fruchtfleisch und einen sehr harten Samen mit zwei oder mehr ölreichen Kernen (bis zu 56 % Öl), aus denen das Marulaöl gewonnen wird. Das Fettsäuregemisch des Marulaöls (Sclerocarya Birrea Kernöl) enthält üblicherweise 65,0 - 70,0 Gew.-% Ölsäure, 13,0 - 15,0 Gew.-% Palmitinsäure, 9,0 - 11,0 Gew.-% Stearinsäure, bis zu 8,0 Gew.-% Linolsäure.

Makettisamenöl (Schinzipphyton Rautenii Kernöl) wird alternativ auch als Mongongo-Öl bezeichnet. Das Öl wird aus dem Kern der Frucht von Mongongo durch übliche Pressverfahren gewonnen. Sonnenblumenöl hat üblicherweise eine Fettsäurezusammensetzung von max. 0,1 Gew.-% Laurinsäure, max. 0,2 Gew.-% Myristinsäure, 4,0 - 8,0 Gew.-% Palmitinsäure, max. 0,3 Gew.-% Palmitoleinsäure, 3,0 - 7,0 Gew.-% Stearinsäure, 14,0 - 39,4 Gew.-% Ölsäure, 60,0 - 88,0 Gew.-% Linosläure, max. 0,3 Gew.-% Linolensäure, max. 0,5 gew.-% Arachinsäure, max. 0,3 Gew.-% Gadoleinsäure und 0,3 - 1,5 Gew.-% Behensäure.

Olivenöl ist aus dem Fruchtfleisch und aus dem Kern von Oliven gepresstes Pflanzenöl. In Olivenöl finden sich durchschnittlich Fettsäuren als Mischung aus 64,0 - 68,0 gew.-% Gew.-% Ölsäure, 11,0 - 16,0 Gew.-% Linolsäure, 8,0 - 10,0 Gew.-% Palmitinsäure, 4,0 - 6,0 Gew.-% Eicosaensäure und 4,0 - 6,0 Gew.-% Palmitoleinsäure.

Hagebuttenkernöl (Rosa Rubiginosa Kernöl) wird aus den in den Hagebutten, den Früchten der Rosen, enthaltenen Samen durch mechanisches Pressen oder über eine Extraktion gewonnen. Die Fettsäurezusammensetzung des Hagebuttenkernöls liegt durchschnittlich bei 27,0 - 31,0 Gew.-% Ölsäure, 55,0 - 58,0 Gew.-% Linolsäure, 8,0 - 10,0 Gew.-% Linolensäure, 2,0 - 6,0 Gew.-% Palmitinsäure und 1,5 - 4,0 Gew.-% Stearinsäure.

Distelkernöl oder Distelsamenöl besitzt eine typische Fettsäurezusammensetzung von 5,3 - 8,0 Gew.-%, Palmitinäsure, 1,9 - 2,9 gew.-% Stearinsäure, 8,4 - 21,3 Gew.-% Ölsäure, 67,8 - 83,2 Gew.-% Linolsäure und max. 0,1 Gew.-% Linolensäure.

Jedes Pflanzenöl ist durch seinen Gehalt an verschiedenen Fettsäuren in spezifischen Mengenbereichen charakterisiert. Da es sich bei den Pflanzenölen um natürliche Inhaltsstoffe handelt, unterliegen die angegebenen Fettsäuremengen einer natürlichen Schwankungsbreite, die je nach Herkunft des Öls und den jeweils herrschenden Umweltbedingungen leicht variieren kann. Für alle im jeweiligen Öl enthaltenen Fettsäuren sind daher die üblichen Mengenbereiche angegeben. Selbstverständlich ergänzen sich in jeder speziellen Öl-Charge alle angegebenen prozentualen Gewichtsmengen zu maximal 100 Gew.-%.

Innherhalb der Gruppe der vornannten Fettsäuremischungen (F1) haben sich Gemische aus Fettsäuren, die in ihrer Zusammensetzungen ganz speziellen Pflanzenölen entsprechen, als explizit besonders bevorzugt erwiesen. Als besonders vorteilhaft hat es sich herausgestellt, wenn es sich bei der Fettsäuremischung (F1) um ein Gemisch aus Fettsäuren handelt, das in seiner Zusammensetzung der Fettsäurezusammensetzung eines Öls aus der Gruppe aus Aprikosenkernöl, Arganöl, Avocadoöl oder Sonnenblumenöl, besonders bevorzugt Sonnenblumenöl, entspricht. Fettsäuremischungen (F1), die in ihrer Zusammensetzungen den vorgenannten Pflanzenölen entsprechen, führten - bei ihrem Einsatz in Form der Estergemische (A) und (B)- zu einer besonders hohen Konditionierwirkung des Shampoos, die sich in besonders geschmeidigem Griff und besonders guter Nass- und Trocken-Kämmbarkeit äußerte.

Weiter ist das hier offenbarte konditionierende Shampoo dadurch gekennzeichnet, dass es sich bei der Fettsäuremischung (F1) um ein Gemisch aus Fettsäuren handelt, das in seiner Zusammensetzung der Fettsäurezusammensetzung eines Öls aus der Gruppe aus Aprikosenkernöl, Arganöl, Avocadoöl oder Sonnenblumenöl, besonders bevorzugt von Sonnenblumenöl, entspricht.

In der explizit am allermeisten bevorzugten Ausführungsform ist das erfindungsgemäße konditionierende Shampoo dadurch gekennzeichnet, dass es sich bei der Fettsäuremischung (F1) um ein Gemisch aus Fettsäuren handelt, das in seiner Zusammensetzung der Fettsäurezusammensetzung von Sonnenblumenöl, entspricht.

Wie bereits zuvor beschrieben ist die Fettsäuremischung (F1), die in ihrer Zusammensetzung der Fettsäurezusammensetzung von Sonnenblumenöl entspricht, gekennzeichnet durch die Hauptbestandteile

4,0 - 8,0 Gew.-% Palmitinsäure, und
3,0 - 7,0 Gew.-% Stearinsäure, und
14,0 - 39,4 Gew.-% Ölsäure, und
60,0 - 88,0 Gew.-% Linolsäure.

Auch hier ergänzen sich alle genannten Gewichtsanteile zu maximal 100 Gew.-%, wobei in kleinen Mengen zusätzlich auch noch eine oder mehrere weitere der vorgenannten Fettsäuren enthalten sein können.

In einer weiteren besonders bevorzugten Ausführungsform ist das erfindungsgemäße konditionierende Shampoo dadurch gekennzeichnet, dass es sich bei der Fettsäuremischung (F1) um ein Gemisch aus Fettsäuren handelt, das - bezogen auf das Gesamtgewicht der Fettsäuremischung (F1) -

4,0 - 8,0 Gew.-% Palmitinsäure, und
3,0 - 7,0 Gew.-% Stearinsäure, und
14,0 - 39,4 Gew.-% Ölsäure, und
60,0 - 88,0 Gew.-% Linolsäure umfasst.

Aus dieser ganz besonders bevorzugten Fettsäuremischung (F1) und Glycerin wird dann durch Versterung mit Glycerin das Gemisch (A) aus den Mono-, Di- und Tri-Estern dieser Fettsäuremischung (F1) und Glycerin ausgebildet. Mit anderen Worten beinhaltet dann die Summe aller Mono-, Di- und Tri-Ester des Gemisches (A) eine Fettsäuremischung (F1) mit einer Verteilung, die - bezogen auf das Gesamtgewicht der Fettsäuremischung -

4,0 - 8,0 Gew.-% Palmitinsäure, und
3,0 - 7,0 Gew.-% Stearinsäure, und
14,0 - 39,4 Gew.-% Ölsäure, und
60,0 - 88,0 Gew.-% Linolsäure umfasst

Aus dieser ganz besonders bevorzugten Fettsäuremischung (F1) und den Polyethylenglycolen mit einer mittleren Molmasse von 200 bis 800 g/mol das wird dann durch Veresterung das Gemisch der Mono- und Dieester (B) ausgebildet. Mit anderen Worten beinhaltet dann die Summe aller Mono- und Di- Ester des Gemisches (B) eine Fettsäuremischung (F1) mit einer Verteilung, die - bezogen auf das Gesamgewicht der Fettsäuremischung -

4,0 - 8,0 Gew.-% Palmitinsäure, und
3,0 - 7,0 Gew.-% Stearinsäure, und
14,0 - 39,4 Gew.-% Ölsäure, und
60,0 - 88,0 Gew.-% Linolsäure umfasst.

Ein konditionierendes Shampoos mit besonders klarer Optik und guter Pflegewirkung kann erhalten werden, wenn das Shampoo das Gemisch der Mono-, Di- und Tri-Ester (A) und das Gemisch der Mono- und Diester (B) in bestimmten Mengenbereichen enthält.

Wenn das Shampoo die Gemische (A) und (B) - bezogen auf das Gesamtgewicht des Shampoos - in einer Gesamtmenge [(A) + (B)] von 0,4 bis 3,5 Gew.-%, bevorzugt von 0,5 bis 2,5 Gew.-%, weiter bevorzugt von 0,6 bis 2,0 Gew.-%, noch weiter bevorzugt von 0,7 bis 1,5 Gew.-% und ganz besonders bevorzugt von 0,8 bis 1,4 Gew.-% enthält, können transparente Formulierungen erzielt werden, die auf dem shampoonierten Haar einen besonders angenehmen Griff erzeugen. Mit diesen Mitteln behandelte Haare zeichnen sich darüber hinaus durch eine besonders gute Nass- und Trockenkämmbarkeit aus. Wenn die Gemische (A) und (B) in einer Gesamtmenge oberhalb von 3,5 Gew.-% im Shampoo eingesetzt werden, läuft die Konditionierwirkung gegen einen Grenzwert, d.h. der Einsatz noch höherer Gesamtmengen [(A)+(B)] führt dies nicht zu einer noch weiteren Steigerung der Pflegeleistung.

In einer weiteren besonders bevorzugten Ausführungsform ist das erfindungsgemäße konditionierende Shampoo dadurch gekennzeichnet, dass es - bezogen auf das Gesamtgewicht des Shampoos - die Gemische (A) und (B) in einer Gesamtmenge [(A) + (B)] von 0,4 bis 3,5 Gew.-%, bevorzugt von 0,5 bis 2,5 Gew.-%, weiter bevorzugt von 0,6 bis 2,0 Gew.-%, noch weiter bevorzugt von 0,7 bis 1,5 Gew.-% und ganz besonders bevorzugt von 0,8 bis 1,4 Gew.-% enthält.

Die Gemische (A) und (B) sind beispielsweise unter den INCI-Bezeichnungen Olivenöl PEG-8 Ester, Aprikosenkernöl PEG-8 Ester, Arganöl PEG-8 Ester, Avocadoöl PEG-8 Ester oder Sonnenblumen-öl PEG-8 Ester bekannt.

Olivenöl PEG-8 Ester kann beispielsweise durch Umesterung von Olivenöl mit PEG-8 (ein Gemisch aus Polyethylenglycolen mit einem mittleren Molgewicht von 370 g/mol) erhalten werden. Olivenöl PEG-8 Ester ist ein Rohstoff, der sowohl das Gemisch (A) als auch das Gemisch (B) enthält.

Aprikosenkernöl PEG-8 Ester kann beispielsweise durch Umesterung von Aprikosenkernöl mit PEG-8 (ein Gemisch aus Polyethylenglycolen mit einem mittleren Molgewicht von 370 g/mol) erhalten werden. Aprikosenkernöl PEG-8 Ester ist ein Rohstoff, der sowohl das Gemisch (A) als auch das Gemisch (B) enthält.

Analog kann Arganöl PEG-8 Ester durch Umesterung von Arganöl mit PEG-8 erhalten werden. Avocadoöl PEG-8 Ester durch Umesterung von Avocadoöl mit PEG-8 erhalten werden. Arganöl PEG-8 Ester ist ein Rohstoff, der sowohl das Gemisch (A) als auch das Gemisch (B) enthält. Sonnenblumen-öl PEG-8 Ester kann durch Umesterung von Sonnenblumenöl mit PEG-8 erhalten werden. Sonnenblumenöl PEG-8 Ester ist ein Rohstoff, der sowohl das Gemisch (A) als auch das Gemisch (B) enthält.

Das offenbarte konditionierende Shampoo enthält bevorzugt Sonnenblumenöl PEG-8 Ester, INCI-Bezeichnung.

Die erfindungsgemäßen konditionierenden Shampoos zeichnen sich durch ihre gute Pflegewirkung aus, wobei sie das Haar konditionieren, ohne beschwerend zu wirken. Auch nach wiederholter Anwendung der erfindungsgemäßen Shampoos tritt keine "Überpflegung" auf, d.h. der Anwender beobachtet auch nach mehrmaligem Shampooneren keinen fettigen Glanz, kein schmieriges Haargefühl und keine Volumenverringerung.

In diesem Zusammenhang ist erfindungswesentlich, dass die Shampoos neben dem Gemisch (A) aus den Mono-, Di- und Tri-Estern der Fettsäuremischung (F1) und Glycerin, im wesentlichen frei sind von anderen Pflanzenölen. Mit anderen Worten ist es zur Vermeidung der Überpflege von zentraler Bedeutung, dass der Tri-Ester der Fettsäuremischung (F1) und Glycerin nur in Abmischung mit den entsprechenden Mono- und Diestern derselben Fettsäuremischung im Shampoo enthalten ist, dass andere Pflanzenöle dem Shampoo jedoch nicht zugesetzt werden.

Aus diesem Grund ist es ein erfindungswesentliches Merkmal der konditionierenden Shampoos, dass die Gesamtmenge aller im Shampoo enthaltenen Pflanzenöle, die von den Tri-Estern der Fettsäuremischung (F1) und Glycerin verschieden sind, bei einem Wert von maximal 0,25 Gew.-% liegt. Hierbei ist die prozentuale Gewichtsangabe auf das Gesamtgewicht das Shampoos bezogen. Wie bereits zuvor beschrieben handelt es sich bei einem Pflanzenöl im Sinne der vorliegenden Erfindung um ein Gemisch von Fettsäuretriglyceriden pflanzlicher Herkunft, dass bei Raumtemperatur (22 °C) und Normaldruck (1 atm bzw. 1013 mbar) wachsartig bis flüssig, bevorzugt flüssig ist.

Liegt der Gehalt an zusätzlich zugesetzten Pflanzenölen (d.h. von Pflanzenölen, die von den Tri-Estern der Fettsäuremischung (F1) und Glycerin verschieden sind) bei maximal 0,25 Gew.-%, so kann die Überpflege des Haares vermieden werden. Auch ist die Formulierung eines Shampoos mit klarer Optik möglich. Bevorzugt liegt der Gehalt an zusätzlichen Pflanzenölen jedoch bei einem Wert von maximal 0,20 Gew.-%, weiter bevorzugt bei maximal 0,15 Gew.-%, noch weiter bevorzugt bei maximal 0,10 Gew.-% und ganz besonders bevorzugt bei maximal 0,05 Gew.-%, wobei alle vorangegangenen prozentualen Gewichtsangaben jeweils auf das Gesamtgewicht des Shampoos bezogen sind. Je geringer der Gehalt an zusätzlichen Olen ist, desto besser kann die "Überpflege" der Haare vermieden werden.

In einer weiteren besonders bevorzugten Ausführungsform ist das erfindungsgemäße konditionierende Shampoo dadurch gekennzeichnet, dass - bezogen auf das Gesamtgewicht des Shampoos - die Gesamtmenge aller im Shampoo enthaltenen Pflanzenöle, die von den Tri-Estern der Fettsäuremischung (F1) und Glycerin verschieden sind, bei einem Wert von maximal 0,20 Gew.-%, bevorzugt von maximal 0,15 Gew.-%, weiter bevorzugt von maximal 0,10 Gew.-% und ganz besonders bevorzugt von maximal 0,05 Gew.-% liegt.

Überraschenderweise hat sich herausgestellt, dass die Einarbeitung der Gemische aus (A) und (B) in die Shampoos in einer genauso guten bzw. sogar höheren Konditionierwirkung resultiert. Im Vergleich zu Shampoos, die nicht die Gemische (A) und (B), sondern stattdessen dieselbe Menge eines analogen Pflanzenöls enthielten, wurde hierbei eine mindestens genauso gute bzw. bessere Kontitionierwirkung erzielt, ein Beschweren und ein fettiges Aussehen des Haares jedoch vermieden. Als fakultativen Bestanteil kann das erfindungsgemäße Shampoo prinzipiell mineralische Öle enthalten. Als mineralische Öle kommen beispielsweise Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).

In einer weiteren bevorzugten Ausführungsform ist das erfindungsgemäße konditionierende Shampoo jedoch dadurch gekennzeichnet, dass - bezogen auf das Gesamtgewicht des Shampoos - die Gesamtmenge aller im Shampoo enthaltenen mineralischen Öle aus der Gruppe der Kohlenwasserstoffe bei einem Wert von maximal 0,25 Gew.-%, bevorzugt bei maximal 0,20 Gew.-%, weiter bevorzugt bei maximal 0,15 Gew.-% und ganz besonders bevorzugt bei maximal 0,05 Gew.-% liegt.

Als weiteren fakultativen Bestandteil kann das erfindungsgemäße Shampoo auch weitere Fettstoffe enthalten. Unter Fettstoffen sind zu verstehen Fettalkohole sowie natürliche und synthetische Wachse, welche sowohl in fester Form als auch flüssig in wässriger Dispersion vorliegen können. Fettsäuren werden im Sinne der vorliegenden Erfindung nicht als Fettstoffe, sondern als anionische Tenside betrachtet.

Als Fettalkohole können beispielsweise gesättigte, ein- oder mehrfach ungesättigte, verzweigte oder unverzweigte Fettalkohole mit Cs - $C_{30}$-, bevorzugt $C_{10}$ - $C_{22}$- und ganz besonders bevorzugt $C_{12}$ - $C_{22}$- Kohlenstoffatomen genannt werden. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol.

In einer weiteren besonders bevorzugten Ausführungsform ist das erfindungsgemäße konditionierende Shampoo jedoch dadurch gekennzeichnet, dass - bezogen auf das Gesamtgewicht des Shampoos - die Gesamtmenge aller im Shampoo enthaltenen $C_8$-$C_{30}$-Fettalkohole bei einem Wert von maximal 0,25 Gew.-%, bevorzugt bei maximal 0,20 Gew.-%, weiter bevorzugt bei maximal 0,15 Gew.-% und ganz besonders bevorzugt bei maximal 0,05 Gew.-% liegt.

Als weiteren prinzipiell einsetzbaren Fettstoff kann das erfindungsgemäße Shampoo auch einen Dialkylether enthalten. Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyln-octylether und 2-Methylpentyl-n-octylether. Besonders bevorzugt ist der Di-n-octylether, der im Handel unter der Bezeichnung Cetiol® OE erhältlich ist.

In einer weiteren besonders bevorzugten Ausführungsform ist das erfindungsgemäße konditionierende Shampoo jedoch dadurch gekennzeichnet, dass - bezogen auf das Gesamtgewicht des Shampoos - die Gesamtmenge aller im Shampoo enthaltenen Di-n-($C_{12}$-$C_{36}$)-alkylether bei einem Wert von maximal 0,25 Gew.-%, bevorzugt bei maximal 0,20 Gew.-%, weiter bevorzugt bei maixmal 0,15 Gew.-% und ganz besonders bevorzugt bei maximal 0,05 Gew.-% liegt.

Konditionierende Shampoos, die wie zuvor beschrieben ohne nennenswerte Anteile an Fettstoffen formuliert werden können, besitzen den zusätzlichen Vorteil, dass ihre Herstellung als klare, transparente Formulierung möglich ist.

Auch Silikone besitzen zwar wie Öle eine Konditionierwirkung, beschweren jedoch ebenfalls das Haar, bilden oftmals einen glänzenden Film aus und erzeugen auf diese Weise einen optischen Eindruck, der bereits das frisch gewaschene Haar fettig erscheinen lässt.

Aus diesem Grund ist es ein weiteres erfindungswesentliches Merkmal des konditionierenden Shampoos, dass die Gesamtmenge aller im Shampoo enthaltenen Silikon-Verbindungen bei einem Wert von maximal 0,25 Gew.-% liegt. Hierbei ist die prozentuale Gewichtsangabe auf das Gesamtgewicht das Shampoos bezogen.

Der Begriff "Silikone" ist eine Bezeichnung für eine Gruppe synthetischer Polymere, in denen Siliciumatome über Sauerstoffatome verknüpft vorliegen. Silikone werden auch als Poly(organo)-siloxane bezeichnet. Unter dem Begrifft "Silikon" wird im Sinne der vorliegenden Erfindung jede Substanz verstanden, deren Molekülstruktur mindestens eine Silicium-Atom (Si-Atom) umfasst.

Wenn der Gehalt an Silikonen im erfindungsgemäßen konditionierenden Shampoo bei maximal 0,25 Gew.-% liegt, kann eine Filmbildung und ein fettiges Aussehen der Haare vermieden werden. Bevorzugt liegt die Gesamtmenge aller im Shampoo enthaltener Silikone - bezogen auf das Gesamtgewicht des Shampoos - jedoch bei einem Wert von maximal 0,20 Gew.-%, bevorzugt bei maximal 0,15 Gew.-%, weiter bevorzugt bei maximal 0,10 Gew.-% und ganz besonders bevorzugt bei maximal 0,01 Gew.-%.

In einer weiteren besonders bevorzugten Ausführungsform ist das erfindungsgemäße konditionierende Shampoo dadurch gekennzeichnet, dass - bezogen auf das Gesamtgewicht des Shampoos - die Gesamtmenge aller im Shampoo enthaltenen Silikon-Verbindungen bei einem Wert von maximal 0,20 Gew.-%, bevorzugt bei maximal 0,15

Gew.-%, weiter bevorzugt bei maximal 0,10 Gew.-% und ganz besonders bevorzugt bei maximal 0,01 Gew.-% liegt.

Silikone sind beispielsweise die folgenden Verbindungen:

(i) Polyalkylsiloxane, Polyarylsiloxane, Polyalkylarylsiloxane, die flüchtig oder nicht flüchtig, geradkettig, verzweigt oder cyclisch, vernetzt oder nicht vernetzt sind;
(ii) Polysiloxane, die in ihrer allgemeinen Struktur eine oder mehrere organofunktionelle Gruppen enthalten, die ausgewählt sind unter:

a) substituierten oder unsubstituierten aminierten Gruppen;
b) (per)fluorierten Gruppen;
c) Thiolgruppen;
d) Carboxylatgruppen;
e) hydroxylierten Gruppen;
f) alkoxylierten Gruppen;
g) Acyloxyalkylgruppen;
h) amphoteren Gruppen;
i) Bisulfitgruppen;
j) Hydroxyacylaminogruppen;
k) Carboxygruppen;
l) Sulfonsäuregruppen; und
m) Sulfat- oder Thiosulfatgruppen;

(iii) linearen Polysiloxan(A)- Polyoxyalkylen(B)- Blockcopoylmeren vom Typ $(A-B)_n$ mit n > 3;
(iv) gepfropfte Siliconpolymere mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Die vorgenannten sowie alle weiteren Silikone sind im erfindungsgemäßen Shampoo - bezogen sein Gesamtgewicht - in einer Gesamtmenge von maximal 0,25 Gew.-% enthalten.

Als weitere Komponente können die erfindungsgemäßen Shampoos optional auch (C) PEG-40 Hydrogenated Castor Oil enthalten. PEG-40 hydrogenated Castor Oil besitzt die CAS-Nr. 61788-85-0 und kann beispielsweise unter dem Handelsnamen Eumulgin CO 40 (BASF) kommerziell erworben werden.

PEG-40 Hydrogenated Castor Oil wird in Shampoos oft als typischer Lösungsvermittler eingesetzt. Lösungsvermittler üben in der Regel einen großen Einfluss auf die Viskosität des Shampoos aus, d.h. je mehr Lösungsvermittler im Shampoo enthalten ist, desto geringer wird in die Viskosität des Shampoos. Für das Einarbeiten und Stabilisieren von konventionellen Pflanzenölen in Shampoos werden üblicherweise große Mengen an Lösungsvermittler benötigt.

Im Zuge der zu dieser Erfindung führenden Arbeiten hat sich nun herausgestellt, dass die erfindungsgemäßen Gemische (A) und (B) sich sehr gut auch ohne den Einsatz an großen Mengen an Lösungsvermittler in das Shampoo einarbeiten lassen.

Überraschenderweise beeinflussen die Gemische (A) und (B) die Viskosität des Shampoos nicht so stark wie PEG-40 hydrogenated Castor Öil (C).

Durch Einsatz der Komponenten (A), (B) und (C) in ihren optimalen, aufeinander abgestimmten Mengenbereichen konnte so ein Shampoo hergestellt werden, dessen Viskosität sich zuverlässig auf den gewünschten Bereich einstellen ließ, und dessen Viskosität auch über lange Zeiträume stabil blieb. Besonders stabile, Formulierungen, deren Viskositäten sich weder bei längerer Lagerung noch bei Temperaturschwankungen wesentlich veränderten, konnten erhalten werden, wenn als Gewichtsverhältnis [(A)+(B)]/(C) ein Wert gewählt wurde, der im Bereich von 4,0 bis 20,0, bevorzugt von 6,0 bis 16,0, und besonders bevorzugt von 8,0 bis 14,0 liegt.

In einer weiteren besonders bevorzugten Ausführungsform ist das erfindungsgemäße konditionierende Shampoo dadurch gekennzeichnet, dass es zusätzlich (C) PEG-40 Hydrogenated Castor Oil enthält, wobei das Gewichtsverhältnis von [(A)+(B)]/(C) bei einem Wert von 4,0 bis 20,0, bevorzugt von 6,0 bis 16,0, und besonders bevorzugt von 8,0 bis 14,0 liegt.

Beispiel: 100 g eines konditionierendes Shampoo enthalten neben weiteren Formulierungsbestandteilen

- 1,0 g Sonnenblumen-öl PEG-8 Ester (Gemische (A) und (B)) und

- 0,1 g PEG-40 hydrogenated Castor Oil

Das Gewichtsverhältnis [(A)+(B)]/(C) liegt bei (1,0 g/ 0,1 g) = 10,0.

Bedingt durch den Einsatz der Gemische (A) und (B) sowie gegebenenfalls des PEG-40 Hydrogenated Castor Oils (C) in ihren aufeinander abgestimmten Gewichtsverhältnissen kann auf diesem Wege ein im Hinblick auf seine Viskosität optimal eingestelltes Shampoo formuliert werden. Auf den Einsatz weiterer Verdicker kann hierbei verzichtet werden.

Insbesondere kann in diesem Zusammenhang auch auf den Einsatz von Verdickern aus der Gruppe der anionischen Polymere auf Basis von (Meth)acrylsäure verzichtet werden.

Anionische (Meth)acrylsäure Polymere sind synthetische Polymere, die durch Polymerisation oder Copolymerisation von Acryläure und/oder Methyacryläure-Monomeren erhalten werden.

In einer weiteren besonders bevorzugten Ausführungsform ist das erfindungsgemäße konditionierende Shampoo dadurch gekennzeichnet, dass - bezogen auf das Gesamtgewicht des Shampoos -
die Gesamtmenge aller im Shampoo enthaltenen anionischen Polymere auf Basis von (Meth)acrylsäure bei einem Wert von maximal 0,25 Gew.-%, bevorzugt bei maximal 0,20 Gew.-%, weiter bevorzugt bei maximal 0,10 Gew.-% und besonders bevorzugt bei maximal 0,01 Gew.-% liegt. Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen.

Synthetische Polymere werden durch Polymerisation eines Monomertyps oder durch Polymerisation von verschiedenen, strukturell voneinander unterschiedlichen Monomertypen hergestellt. Wird das Polymer durch Polymerisation eines Monomertyps hergestellt, spricht man von Homo-Polymeren. Werden strukturell unterschiedliche Monomertypen in der Polymerisation eingesetzt, wird das resultiertende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des kationischen Polymers (d) nicht mehr als $10^7$ g/mol, bevorzugt nicht mehr als $10^6$ g/mol und besonders bevorzugt nicht mehr als $10^5$ g/mol beträgt

Unter einem anionischen Polymer auf Basis von (Methy)acrylsäure wird im Sinne der vorliegenden Erfindung ein Polymer verstanden, das mindestens eine Wiederholungseinheit der Formel (PI) und/oder mindestens eine Struktureinheit der Formel (PII) umfasst.

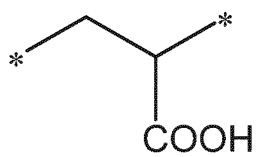 (PI) 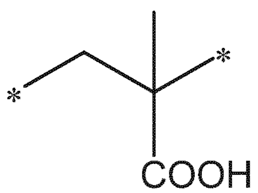 (PII)

In wässriger Lösung liegt ein Großteil der Carboxygruppen der Struktureinheiten der Formel (PI) bzw. (PII) in deprotonierter und damit anionischer Form vor.

Unter den Polyacryl- und Polymethacryl-Polymeren sind vernetzte oder unvernetzte Polyacrylsäure- und/oder Polymethacrylsäure-Polymere zu verstehen, wie sie beispielsweise von der Firma 3V Sigma unter den Handelsnamen Synthalen K oder Synthalen M oder von der Firma Lubrizol unter den Handelsnamen Carbopol (beispielsweise Carbopol 980, 981, 954, 2984, 5984 und/oder Silk 100), jeweils mit der INCI-Bezeichnung Carbomer, erhältlich ist. Auch das von der BASF vertriebene unter dem Handelsnamen Cosmedia SP (INCI Name: SODIUM POLYACRYLATE) bekannte Produkt kann in diesem Zusammenhang als bevorzugtes Acrylsäure-Homopolymer genannt werden. Als Polyacryl- und Polymethacryl-Polymere können auch Copolymere der Acrylsäure und/oder der Methacrylsäure eingesetzt werden. Ein in diesem Zusammenhang zu nennendes Polymer ist das unter der INCI Bezeichnung Acrylates/C10-30 Alkyl Acrylate Crosspolymer bekannte Polymer, das unter dem Handelsnamen Carbopol 1382 von der Firma Noveon erhältlich ist. Ein weiterhin zu nennendes Polymer ist das unter der INCI-Bezeichnung Acrylates/Steareth-20 Methacrylate Crosspolymer bekannte Polymer, welches beispielsweise mit dem Handelsnamen Aculyn® 88 von der Firma Rohm & Haas vertrieben wird. Ferner können Polymere mit der INCI-Nomenklatur Acrylates/Palmeth-25 Acrylate Copolymer oder Acrylates/Palmeth-20 Acrylate Copolymer genannt werden. Solche Polymere sind beispielsweise unter der Handelsbezeichnung Synthalen® W 2000 als von der Firma 3 V Sigma erhältlich.

Es ebenfalls möglich, ein Copolymer aus mindestens einem anionischen Acrylsäure bzw. Methacrylsäure-Monomer und mindestens einem nichtionogenen Monomer einzusetzen. Mögliche nichtionogene Monomere sind in diesem Zusammenhang Acrylamid, Methacrylamid, Acrylsäureester, Methacrylsäureester, Vinylpyrrolidon, Vinylether und Vinylester.

Weiterhin einsetzbare Polyacryl- und Polymethacryl-Polymere sind beispielsweise Copolymere aus Acrylsäure und/oder Methacrylsäure und deren $C_1$-$C_6$-Alkylestern, wie sie unter der INCI-Deklaration Acrylates Copolymer vertrieben werden. Ein gängiges Handelsprodukt ist beispielsweise Aculyn® 33 der Firma Rohm & Haas. Weiterhin zu nennen sind aber auch Copolymere aus Acrylsäure und/oder Methacrylsäure, den $C_1$-$C_6$-Alkylestern von Acrylsäure und/oder Methacacrylsäure sowie den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn® 22 (INCI-Name: Acrylates/Steareth-20 Methacrylate Copolymer) vertrieben.

All diese (Co)Polymere auf Basis von (Meth)acryläsure werden bevorzugt nur in sehr geringen Mengen im erfindungsgemäßen Shampoo eingesetzt, und besonders bevorzugt wird auf den Einsatz dieser Polymere verzichtet.

Als weiterer üblicher Verdicker wird in Shampoos oft auch Natriumchlorid eingesetzt. Auch der Einsatz von Natriumchlorid kann in den erfindungsgemäßen Shampoos durch den Einsatz der Gemische (A) und (B) sowie gegebenenfalls (C) stark reduziert bzw. vermieden werden.

In einer weiteren besonders bevorzugten Ausführungsform ist das erfindungsgemäße konditionierende Shampoo dadurch gekennzeichnet, dass - bezogen auf das Gesamtgewicht des Shampoos -
die Gesamtmenge des im Shampoo enthaltenen Natriumchlorids bei einem Wert von maximal 0,25 Gew.-%, bevorzugt bei maximal 0,20 Gew.-%, weiter bevorzugt bei maximal 0,10 Gew.-% und besonders bevorzugt bei maximal 0,01 Gew.-% liegt.

Als weitere übliche Verdicker werden in Shampoos oft auch Polysaccharide eingesetzt. Auch der Einsatz von der Plysaccharide kann in den erfindungsgemäßen Shampoos durch den Einsatz der Gemische (A) und (B) sowie gegebenenfalls (C) stark reduziert bzw. vermieden werden.

In einer weiteren besonders bevorzugten Ausführungsform ist das erfindungsgemäße konditionierende Shampoo dadurch gekennzeichnet, dass - bezogen auf das Gesamtgewicht des Shampoos -
die Gesamtmenge aller im Shampoo enthaltenen anionischen und nichtionischen Polysaccharide bei einem Wert von maximal 0,25 Gew.-%, bevorzugt bei maximal 0,20 Gew.-%, weiter bevorzugt bei maximal 0,10 Gew.-% und besonders bevorzugt bei maximal 0,01 Gew.-% liegt.

Der pH-Wert der erfindungsgemäßen Shampoos wird bevorzugt auf einen Wert im Bereich von 4,0 bis 7,0, bevorzugt von 5,3 bis 6,7, besonders bevorzugt von 5,5 bis 6,5 und insbesondere von 5,7 bis 6,3 eingestellt.

Neben den vorgenannten Tensiden können die erfindugnsgemäßen Shampoos zusätzlich auch ein oder mehrere weitere Co-Tenisde enthalten. Bei der Wahl der Co-Tenside ist darauf zu achten, dass diese mild und schaumstark sind, dass sie die Schaumeigenschaften der anionischen Tenside nicht negativ beeinflussen und/oder dass sie die Schaumeigenschaften der anionischen Tenside verstärken.

Als besonders milde Co-Tenside für die erfindungsgemäßen Haarreinigungsmittel haben sich milde amphotere/zwitterionische und/oder milde nichtionische Tenside erwiesen.

Zu den geeigneten milden amphoteren und/oder zwitterionischen Tensiden, die in den erfindungsgemäßen kosmetischen Haarreinigungsmitteln eingesetzt werden können, zählen beispielsweise eine oder mehrere Verbindungen der nachfolgenden Formeln (I) bis (VII), in denen der Rest R jeweils für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

| | |
|---|---|
| 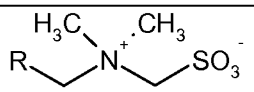 (I) | 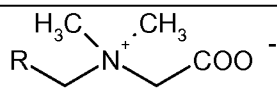 (II) |
| 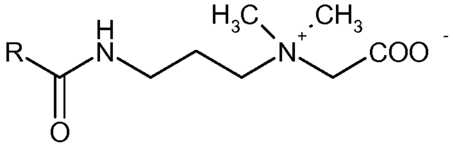 (III) | 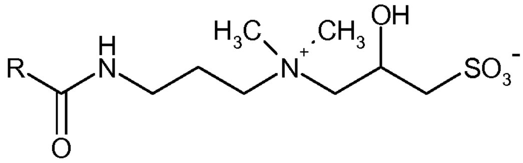 (IV) |
| 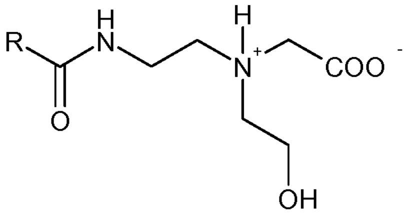 (V) | 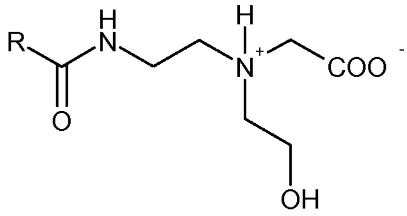 (VI) |
| 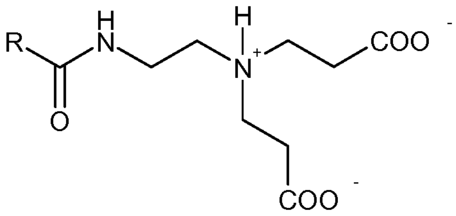 (VII) | |

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 18 und insbesondere mit 10 bis 16 C-Atomen.

Besonders bevorzugt sind amphotere und/oder zwitterionische Tenside, bei denen sich der Rest R von Kokosfett ableitet.

Mehr bevorzugt sind $C_{10}$-$C_{16}$-Alkylampho(di)acetate und/oder $C_{10}$-$C_{16}$-Alkylamido($C_1$-$C_4$)-Alkylbetaine der zuvor genannten Formeln.

Ganz besonders bevorzugt sind die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren/zwitterionischen Tenside.

Insbesondere bevorzugt sind Tenside mit den INCI-Bezeichnungen Cocamidopropylbetain, Lauramidopropylbetain, Cocoampho(di)acetate und/oder Lauroapho(di)acetate.

Der Gewichtsanteil des amphoteren und/oder zwitterionischen Tensids am Gesamtgewicht der erfindungsgemäßen kosmetischen Haarreinigungsmittel beträgt bevorzugt 0,50 bis 15,00 Gew.-%. Mehr bevorzugt ist ein Gewichtsanteil von 1,00 bis 14,00 Gew.-%, besonders bevorzugt von 2,00 bis 13,00 Gew.-%, ganz besonders bevorzugt 2,50 bis 12,50 Gew.-% und insbesondere 3,00 bis 12,00 Gew.-%.

Zu den geeigneten nichtionischen Tensiden, die in den erfindungsgemäßen kosmetischen Haarreinigungsmitteln eingesetzt werden können, zählen

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- Ethylenoxid- und Polyglycerin-Anlagerungsprodukte an Methylglucosid-Fettsäureester, Fettsäurealkanolamide und Fettsäureglucamide,
- Aminoxide,
- Sorbitanfettsäureester und Anlagerungeprodukte von Ethylenoxid an Sorbitanfettsäureester wie beispielsweise Polysorbate,
- Fettsäurealkanolamide der nachfolgenden allgemeinen Formel,

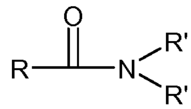

in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe $-(CH_2)_nOH$ stehen, in der n die Zahlen 2 oder 3 bedeutet, mit der Maßgabe, dass mindestens einer der Reste R' für den zuvor genannten Rest $-(CH_2)_nOH$ steht,

- Zuckerfettsäureester und Anlagerungsprodukte von Ethylenoxid an Zuckerfettsäureester,
- Anlagerungsprodukte von Ethylenoxid an Fettsäurealkanolamide und Fettamine, und/oder
- Alkyl(oligo)glucoside,
- Gemische aus Alkyl-(oligo)-glucosiden und Fettalkoholen zum Beispiel das im Handel erhältliche Produkt Montanov®68,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden.

Geeignete Alkyl(oligo)glycoside können ausgewählt sein aus Verbindungen der allgemeinen Formel der $RO-[G]_x$, in denen sich [G] bevorzugt von Aldosen und/oder Ketosen mit 5-6 Kohlenstoffatomen, vorzugsweise von Glucose ableitet.

Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann.

Besonders bevorzugte Alkyl(oligo)glycoside weisen einen Oligomerisierungsgrad zwischen 1,2 und 1,5 auf.

Der Rest R steht bevorzugt für mindestens einen Alkyl- und/oder Alkenylrest mit 4 bis 24 C-Atomen. Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Geeignete Aminoxide können ausgewählt sein aus mindestens einer Verbindung der allgemeinen Formeln (X) oder (Y)

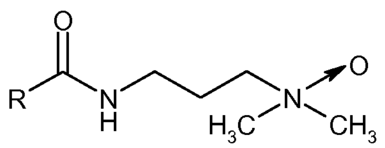
(X)
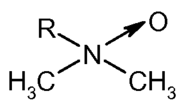
(Y),

in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.

Insbesondere bevorzugt sind die unter den INCI-Bezeichnungen Cocamine Oxide, Lauramine Oxide und/oder Cocamidopropylaminoxid bekannten und im Handel von verschiedenen Anbietern erhältlichen Tenside der zuvor genannten Formel (X) oder (Y).

Besonders bevorzugte nichtionische Tenside, die in den erfindungsgemäßen Zusammensetzungen enthalten sein können, sind Fettsäurealkanolamide, insbesondere die unter den INCI-Bezeichnungen Cocamide MEA und/oder Cocamide MIPA bekannten Verbindungen.

Besonders bevorzugte nichtionische Tenside, die in den erfindungsgemäßen Zusammensetzungen enthalten sein können, sind weiterhin Alkyl(oligo)glucoside, insbesondere die unter den INCI-Bezeichnungen Caprylyl/Capryl Glucoside, Decyl Glucoside, Lauryl Glucoside und/oder Coco Glucoside bekannten Verbindungen;

Der Gewichtsanteil des nichtionischen Tensids am Gesamtgewicht der erfindungsgemäßen kosmetischen Haarreinigungsmittel beträgt bevorzugt 0,10 bis 10,00 Gew.-%. Mehr bevorzugt ist ein Gewichtsanteil von 0,20 bis 8,00 Gew.-%, besonders bevorzugt von 0,30 bis 7,00 Gew.-%, ganz besonders bevorzugt 0,40 bis 6,00 Gew.-% und insbesondere 0,50 bis 5,00 Gew.-%.

Neben den zuvor genannten zwingenden und fakultativen Wirkstoffen kann das erfindungsgemäße Shampoo auch noch einen oder mehrere weitere Wirkstoffe enthalten, die ausgewählt sind aus den

- kationischen Pflegepolymeren
- Proteinhydrolysaten und
- Vitaminen.

Unter geeigneten Proteinhydrolysaten sind Produktgemische zu verstehen, die durch sauer, basisch oder enzymatisch katalysierten Abbau von Proteinen (Eiweißen) erhalten werden können.

Es können Proteinhydrolysate pflanzlichen, tierischen und/oder marinen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Der Gewichtsanteil des oder der Proteinhydrolysats(e) am Gesamtgewicht der erfindungsgemäßen Haarreinigungsmittel beträgt bevorzugt 0,01 bis 3 Gew.-%, mehr bevorzugt 0,025 bis 2 Gew.-% und insbesondere 0,05 bis 1 Gew.-%.

Unter geeigneten Vitaminen sind bevorzugt die folgenden Vitamine, Provitamine und Vitaminvorstufen sowie deren Derivate zu verstehen:

- Vitamin A: zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.

  - Vitamin $B_1$ (Thiamin)
  - Vitamin $B_2$ (Riboflavin)
  - Vitamin $B_3$. Unter dieser Bezeichnung werden häufig die Verbindungen Nicotinsäure und Nicotinsäureamid (Niacinamid) geführt.
  - Vitamin $B_5$ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
  - Vitamin $B_6$ (Pyridoxin sowie Pyridoxamin und Pyridoxal).

- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Insbesondere bevorzugt sind Nicotinsäureamid, Biotin, Pantolacton und/oder Panthenol.

Der Gewichtsanteil des oder der Vitamins(e), Vitaminderivats(e), und/oder der Vitaminvorstufe(n) am Gesamtgewicht der erfindungsgemäßen Haarreinigungsmittel beträgt bevorzugt 0,001 bis 2 Gew.-%, besonders bevorzugt 0,005 bis 1 Gew.-% und insbesondere 0,01 bis 0,5 Gew.-%.

Glycerin kann den erfindungsgemäßen Haarreinigungsmitteln separat in einer Menge von bis zu 10 Gew.-% (bezogen auf das Gesamtgewicht des Mittels) zugegeben werden. Es kann aber auch Bestandteil des zuvor genannten wässrig-alkoholischen Trägers sein.

Durch Einsatz der vorgenannten erfindungswesentlichen Gemische (A) und (B) -gegebenenfalls in Kombination mit (C) - kann in den konditionierenden Shampoos trotz Verzicht auf die sonst üblichen vorgenannten Verdicker eine Viskosität von 7000 bis 12 000 mPas einstellt werden. Die mittels (A), (B) - sowie gegebenenfalls (C) - eingestellten Viskositäten sind herbei gegenüber langen Lagerzeiten und Temperaturschwankungen sehr stabil.

Alle Viskositäten wurden bei 20 °C mit einem Brookfield Viskosimeter unter Verwendung der Spindel Nr. 5 bei 20 Umdrehungen pro Minute gemessen.

In einer weiteren besonders bevorzugten Ausführungsform ist das erfindungsgemäße konditionierende Shampoo dadurch gekennzeichnet, dass es eine Viskosität von 7000 bis 12 000 mPas (20 °C / Brookfield Viskosimeter/ Spindel 5 / 20 U/min) besitzt.

Weitere Wirk-, Hilfs- und Zusatzstoffe, die in den erfindungsgemäßen Shampoos bevorzugt enthalten sein können, sind beispielsweise:

- Pflanzenextrakte,
- Feuchthaltemittel,
- Parfums,
- UV-Filter,
- Strukturanten wie Maleinsäure und Milchsäure,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure, und/oder Basen wie Alkanolamine und/oder Natriumhydroxid,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, $N_2O$, Dimethylether, $CO_2$ und Luft,

- Antioxidantien,
- Konservierungsmittel, wie beispielsweise Natriumbenzoat, Phenoxyethanol und/oder Salicylsäure,

Beispiele:

Es wurden die folgenden konditionierenden Shampoos hergestellt. Die Mengenangaben in den Tabellen beziehen sich auf [Gew.-%]):

| | **V** | **E** |
|---|---|---|
| Natrium Laureth Sulfat (70 %ige wässrige Lsg.) | 12,5 | 12,5 |
| Dinatrium Cocoamphodipropionat | 2,0 | 2,0 |
| Cocoamidopropylbetain (40 %ige wässrige Lsg.) | 4,0 | 4,0 |
| Cocoamid Monoethanolamin | 0,9 | 0,9 |
| Sonnenblumenöl PEG-8 Ester | -- | 1,0 |
| Zitronensäure | 0,5 | 0,5 |
| Natriumbenzoat | 0,5 | 0,5 |
| Polyquaternium-10 | 0,3 | 0,3 |
| PEG-40 Hydrogenated Castor Oil | 0,1 | 0,1 |
| Wasser | ad 100 | ad 100 |
| pH | 4,5-5,0 | 4,5-5,0 |

Die Shampoos des Beispiels V (Vergleich) sowie des Beispiels E (erfindungsgemäß) wurden an jeweils 5 Probanden im Hinblick auf ihre Konditionierwirkung bewertet. Die Bewertung erfolgte von geschulten Personen (Frisöre), denen die Inhaltstoffe der jeweils getesteten Formulieurngen nicht bekannt waren. Die Bewertung erfolgte anhand einer Skala von 1 (sehr schlecht) bis 4 (sehr gut).

Aus allen Einzelwerten wurde jeweils der Mittelwert gebildet

Bewertung der Konditionierwirkung

| | **V** | **E** |
|---|---|---|
| Griff des Schaums im Haar | 1,3 | 2,8 |
| Griff des Haares beim Ausspülen | 1,5 | 3,6 |
| Entwirrbarkeit des Haares | 1,7 | 2,3 |
| Kämmbarkeit des nassen Haares | 1,2 | 2,8 |
| Griff des nassen Haares | 1,2 | 3,2 |
| Kämmbarkeit des trockenen Haares | 1,2 | 2,8 |
| Statische Aufladung des trockenen Haares | 1,4 | 3,3 |
| Griff des trockenen Haares | 1,3 | 4,0 |

## Patentansprüche

**1.** Konditionierendes Shampoo, enthaltend

(A) ein Gemisch aus den Mono-, Di- und Tri-Estern von einer Fettsäuremischung (F1) und Glycerin, und
(B) ein Gemisch aus den Mono- und Di-Estern einer Fettsäuremischung (F1) und einem Polyethylenglycol mit einer mittleren Molmasse von 200 bis 800 g/mol, wobei

- es sich bei der Fettsäuremischung (F1) um ein Gemisch aus Fettsäuren handelt, das in seiner Zusammensetzung der Fettsäurezusammensetzung eines Pflanzenöls entspricht, und das - bezogen auf das Gesamtgewicht der Fettsäuremischung (F1) -

4.0 - 8,0 Gew.-% Palmitinsäure, und
3,0 - 7.0 Gew.-% Stearinsäure, und
14,0 - 39,4 Gew.-% Ölsäure, und
60,0 - 88,0 Gew.-% Linolsäure umfasst.

und - bezogen auf das Gesamtgewicht des Shampoos -
- die Gesamtmenge aller im Shampoo enthaltenen Pflanzenöle, die von den Tri-Estern der Fettsäuremischung (F1) und Glycerin verschieden sind, bei einem Wert von maximal 0,25 Gew.-% liegt, und
- die Gesamtmenge aller im Shampoo enthaltenen Silikon-Verbindungen bei einem Wert von maximal 0,25 Gew.-% liegt.

**2.** Shampoo nach Anspruch 1, **dadurch gekennzeichnet, dass** es sich bei dem (A) Gemisch aus den Mono-, Di- und Tri-Estern von der Fettsäuremischung (F1) und Glycerin um ein Gemisch aus den Verbindungen der Gruppen (A1), (A2) und (A3) handelt,

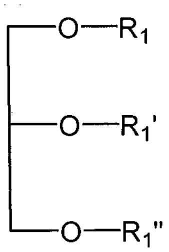 (A1) 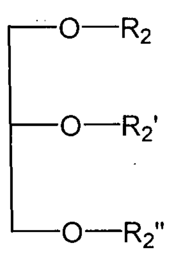 (A2) 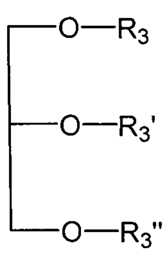 (A3)

wobei

- in jeder der Verbindungen aus der Gruppe (A1) die Reste R1, R1' und R1" unabhängig voneinander für eine lineare, gesättigte oder einfach oder mehrfach ungesättigte $C_8$-$C_{30}$-Acylgruppe stehen, und
- in jeder der Verbindungen aus der Gruppe (A2) einer der Reste aus R2, R2' und R2" für ein Wasserstoffatom steht und die anderen beiden Reste unabhängig voneinander für eine lineare, gesättigte oder einfach oder mehrfach ungesättigte $C_8$-$C_{30}$-Acylgruppe stehen, und
- in jeder der Verbindungen aus der Gruppe (A3) zwei der Reste aus R3, R3' und R3" für ein Wasserstoffatom stehen und der dritte Rest für eine lineare, gesättigte oder einfach oder mehrfach ungesättigte $C_8$-$C_{30}$-Acylgruppe steht.

**3.** Shampoo nach einem der Ansprüche 1 oder 2, **dadurch gekennzeichnet, dass** es sich bei (B) dem Gemisch aus den Mono- und Di-Estern von der Fettsäuremischung (F1) und einem Polyethylenglycol mit einer mittleren Molmasse von 200 bis 800 g/mol um ein Gemisch aus den Verbindungen der Gruppen (B1) und (B2) handelt,

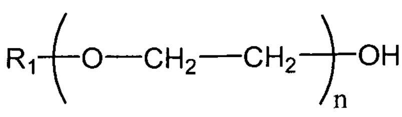 (B1) 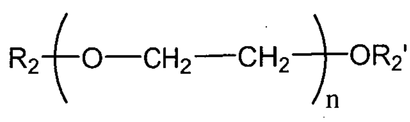 (B2)

wobei

- in jeder der Verbindungen aus der Gruppe (B1) der Rest R1 für eine lineare, gesättigte oder einfach oder mehrfach ungesättigte $C_8$-$C_{30}$-Acylgruppesteht, und
- in jeder der Verbindungen aus der Gruppe (B2) die Reste R2 und R2' unabhängig voneinander für eine lineare, gesättigte oder einfach oder mehrfach ungesättigte $C_8$-$C_{30}$-Acylgruppestehen, und
- in jeder der Verbindungen aus den Gruppen (B1) und (B2) n unabhängig für eine ganze Zahl von 1 bis 50, bevorzugt von 2 bis 40, weiter bevorzugt von von 3 bis 30 und besonders bevorzugt von 4 bis 20 steht.

**4.** Shampoo nach einem der Ansprüche 1 bis 3, **dadurch gekennzeichnet, dass** es sich bei der Fettsäuremischung

(F1) um ein Gemisch aus Fettsäuren handelt, das in seiner Zusammensetzung der Fettsäurezusammensetzung von Sonnenblumenöl, entspricht.

5. Shampoo nach einem der Ansprüche 1 bis 4, **dadurch gekennzeichnet, dass** es - bezogen auf das Gesamtgewicht des Shampoos - die Gemische (A) und (B) in einer Gesamtmenge [(A) + (B)] von 0,4 bis 3,5 Gew.-%, bevorzugt von 0,5 bis 2,5 Gew.-%, weiter bevorzugt von 0,6 bis 2,0 Gew.-%, noch weiter bevorzugt von 0,7 bis 1,5 Gew.-% und ganz besonders bevorzugt von 0,8 bis 1,4 Gew.-% enthält.

6. Shampoo nach einem der Ansprüche 1 bis 6 5, **dadurch gekennzeichnet, dass** es die Gemische (A) und (B) enthält, die unter der INCI-Bezeichnung Sonnenblumenöl PEG-8 Ester bekannt sind.

7. Shampoo nach einem der Ansprüche 1 bis 6, **dadurch gekennzeichnet, dass** - bezogen auf das Gesamtgewicht des Shampoos - die Gesamtmenge aller im Shampoo enthaltenen Pflanzenöle, die von den Tri-Estern der Fettsäuremischung (F1) und Glycerin verschieden sind, bei einem Wert von maximal 0,20 Gew.-%, bevorzugt von maximal 0,15 Gew.-%, weiter bevorzugt von maximal 0,10 Gew.-% und ganz besonders bevorzugt von maximal 0,05 Gew.-% liegt.

8. Shampoo nach einem der Ansprüche 1 bis 7, **dadurch gekennzeichnet, dass** - bezogen auf das Gesamtgewicht des Shampoos - die Gesamtmenge aller im Shampoo enthaltenen Silikon-Verbindungen bei einem Wert von maximal 0,20 Gew.-%, bevorzugt bei maximal 0,15 Gew.-%, weiter bevorzugt bei maximal 0,10 Gew.-% und ganz besonders bevorzugt bei maximal 0,01 Gew.-% liegt.

9. Shampoo nach einem der Ansprüche 1 bis 8, **dadurch gekennzeichnet, dass** es zusätzlich (C) PEG-40 Hydrogenated Castor Oil enthält, wobei das Gewichtsverhältnis von [(A)+(B)]/(C) bei einem Wert von 4,0 bis 20,0, bevorzugt von 6,0 bis 16,0, und besonders bevorzugt von 8,0 bis 14,0 liegt.

10. Shampoo nach einem der Ansprüche 1 bis 9, **dadurch gekennzeichnet, dass** - bezogen auf das Gesamtgewicht des Shampoos - die Gesamtmenge aller im Shampoo enthaltenen anionischen Polymere auf Basis von (Meth)acrylsäure bei einem Wert von maximal 0,25 Gew.-%, bevorzugt bei maximal 0,20 Gew.-%, weiter bevorzugt bei maximal 0,10 Gew.-% und besonders bevorzugt bei maximal 0,01 Gew.-% liegt.

11. Shampoo nach einem der Ansprüche 1 bis 10, **dadurch gekennzeichnet, dass** - bezogen auf das Gesamtgewicht des Shampoos - die Gesamtmenge des im Shampoo enthaltenen Natriumchlorids bei einem Wert von maximal 0,25 Gew.-%, bevorzugt bei maximal 0,20 Gew.-%, weiter bevorzugt bei maximal 0,10 Gew.-% und besonders bevorzugt bei maximal 0,01 Gew.-% liegt.

12. Shampoo nach einem der Ansprüche 1 bis 11, **dadurch gekennzeichnet, dass** - bezogen auf das Gesamtgewicht des Shampoos - die Gesamtmenge aller im Shampoo enthaltenen anionischen und nichtionischen Polysaccharide bei einem Wert von maximal 0,25 Gew.-%, bevorzugt bei maximal 0,20 Gew.-%, weiter bevorzugt bei maximal 0,10 Gew.-% und besonders bevorzugt bei maximal 0,01 Gew.-% liegt.

13. Shampoo nach einem der Ansprüche 1 bis 12, **dadurch gekennzeichnet, dass** es eine Viskosität von 7000 bis 12 000 mPas (20 °C / Brookfield Viskosimeter/ Spindel 5 / 20 U/min) besitzt.

**Claims**

1. A conditioning shampoo containing

   (A) a mixture of the mono-, di- and tri-esters of a fatty acid mixture (F1) and glycerol, and
   (B) a mixture of the mono- and di-esters of a fatty acid mixture (F1) and a polyethylene glycol having an average molar mass of from 200 to 800 g/mol,
   wherein

   - the fatty acid mixture (F1) is a mixture of fatty acids that, in the composition thereof, corresponds to the fatty acid composition of a vegetable oil and that, based on the total weight of the fatty acid mixture (F1), comprises 4.0-8.0 wt.% palmitic acid, 3.0-7.0 wt.% stearic acid, 14.0-39.4 wt.% oleic acid, and 60.0-88.0 wt.% linoleic acid,

and, based on the total weight of the shampoo,
- the total amount of all vegetable oils contained in the shampoo, which oils are different from the tri-esters of the fatty acid mixture (F1) and glycerol, has a value of at most 0.25 wt.%, and
- the total amount of all silicone compounds contained in the shampoo has a value of at most 0.25 wt.%.

2. The shampoo according to claim 1, **characterized in that** the (A) mixture of the mono-, di-and tri-esters of the fatty acid mixture (F1) and glycerol is a mixture of the compounds of groups (A1), (A2) and (A3),

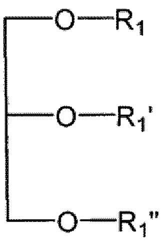 (A1) 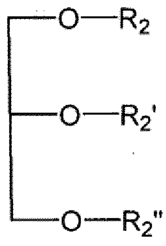 (A2) 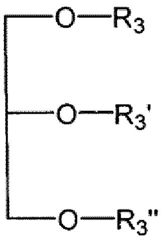 (A3)

wherein,

- in each of the compounds from the group (A1), the functional groups R1, R1' and R1" represent, independently of one another, a linear, saturated or mono- or polyunsaturated $C_8$-$C_{30}$ acyl group, and,
- in each of the compounds from the group (A2), one of the functional groups from R2, R2' and R2" represents a hydrogen atom and the other two functional groups represent, independently of one another, a linear, saturated or mono- or polyunsaturated $C_8$-$C_{30}$ acyl group, and,
- in each of the compounds from the group (A3), two of the functional groups from R3, R3' and R3" represent a hydrogen atom and the third functional group represents a linear, saturated or mono- or polyunsaturated $C_8$-$C_{30}$ acyl group.

3. The shampoo according to one of claims 1 or 2, **characterized in that** the (B) mixture of the mono- and di-esters of the fatty acid mixture (F1) and a polyethylene glycol having an average molar mass of from 200 to 800 g/mol is a mixture of the compounds of groups (B1) and (B2),

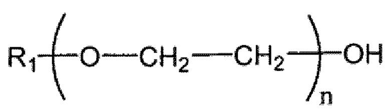 (B1) 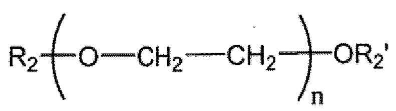 (B2)

wherein,

- in each of the compounds from the group (B1), the functional group R1 represents a linear, saturated or mono- or polyunsaturated $C_8$-$C_{30}$ acyl group, and,
- in each of the compounds from the group (B2), the functional groups R2 and R2' represent, independently of one another, a linear, saturated or mono- or polyunsaturated $C_8$-$C_{30}$ acyl group, and,
- in each of the compounds from the groups (B1) and (B2), n independently represents an integer from 1 to 50, preferably from 2 to 40, more preferably from 3 to 30 and particularly preferably from 4 to 20.

4. The shampoo according to one of claims 1 to 3, **characterized in that** the fatty acid mixture (F1) is a mixture of fatty acids that, in the composition thereof, corresponds to the fatty acid composition of sunflower oil.

5. The shampoo according to one of claims 1 to 4, **characterized in that** it contains, based on the total weight of the shampoo, the mixtures (A) and (B) in a total amount [(A) + (B)] of from 0.4 to 3.5 wt.%, preferably from 0.5 to 2.5 wt.%, more preferably from 0.6 to 2.0 wt.%, even more preferably from 0.7 to 1.5 wt.%, and very particularly preferably from 0.8 to 1.4 wt.%.

6. The shampoo according to one of claims 1 to 5, **characterized in that** it contains the mixtures (A) and (B) which are known under the INCI name Sunflower Seed Oil PEG-8 esters.

7. The shampoo according to one of claims 1 to 6, **characterized in that**, based on the total weight of the shampoo,

the total amount of all vegetable oils contained in the shampoo, which oils are different from the tri-esters of the fatty acid mixture (F1) and glycerol, has a value of at most 0.20 wt.%, preferably at most 0.15 wt.%, more preferably at most 0.10 wt.% and very particularly preferably at most 0.05 wt.%.

**8.** The shampoo according to one of claims 1 to 7, **characterized in that**, based on the total weight of the shampoo, the total amount of all silicone compounds contained in the shampoo has a value of at most 0.20 wt.%, preferably at most 0.15 wt.%, more preferably at most 0.10 wt.% and very particularly preferably at most 0.01 wt.%.

**9.** The shampoo according to one of claims 1 to 8, **characterized in that** it also contains (C) PEG-40 Hydrogenated Castor Oil, the weight ratio of [(A)+(B)]/(C) having a value of from 4.0 to 20.0, preferably from 6.0 to 16.0, and particularly preferably from 8.0 to 14.0.

**10.** The shampoo according to one of claims 1 to 9, **characterized in that**, based on the total weight of the shampoo, the total amount of all anionic polymers on the basis of (meth)acrylic acid that are contained in the shampoo has a value of at most 0.25 wt.%, preferably at most 0.20 wt.%, more preferably at most 0.10 wt.%, and particularly preferably at most 0.01 wt.%.

**11.** The shampoo according to one of claims 1 to 10, **characterized in that**, based on the total weight of the shampoo, the total amount of sodium chloride contained in the shampoo has a value of at most 0.25 wt.%, preferably at most 0.20 wt.%, more preferably at most 0.10 wt.% and particularly preferably at most 0.01 wt.%.

**12.** The shampoo according to one of claims 1 to 11, **characterized in that**, based on the total weight of the shampoo, the total amount of all anionic and nonionic polysaccharides contained in the shampoo has a value of at most 0.25 wt.%, preferably at most 0.20 wt.%, more preferably at most 0.10 wt.% and particularly preferably at most 0.01 wt.%.

**13.** The shampoo according to one of claims 1 to 12, **characterized in that** it has a viscosity of from 7,000 to 12,000 mPas (20 °C / Brookfield viscometer / spindle 5 / 20 rpm).

## Revendications

**1.** Shampooing de conditionnement contenant

(A) un mélange de mono, di et triesters d'un mélange d'acides gras (F1) et de glycérol, et
(B) un mélange de mono- et diesters d'un mélange d'acides gras (F1) et d'un polyéthylèneglycol ayant un poids moléculaire moyen de 200 à 800 g/mol, dans lequel

- le mélange d'acides gras (F1) est un mélange d'acides gras dont la composition correspond à la composition en acides gras d'une huile végétale et qui comprend, par rapport au poids total du mélange d'acides gras (F1), -4,0 à 8,0 % en poids d'acide palmitique et 3,0 à 7,0 % en poids d'acide stéarique et 14,0 à 39,4 % en poids d'acide oléique, et 60,0 à 88,0 % en poids d'acide linoléique,
et, par rapport au poids total du shampooing,
- la quantité totale de toutes les huiles végétales contenues dans le shampooing, à l'exception des triesters du mélange d'acides gras (F1) et du glycérol, est de 0,25 % en poids au maximum, et
- la quantité totale de tous les composés de silicone contenus dans le shampooing ne dépasse pas 0,25 % en poids.

**2.** Shampooing selon la revendication 1, **caractérisé en ce que** (A) le mélange de mono-, di-et triesters du mélange d'acides gras (F1) et de glycérol est un mélange des composés des groupes (A1), (A2) et (A3),

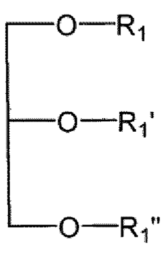
(A1)
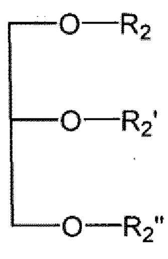
(A2)
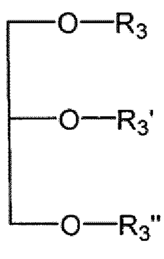
(A3)

dans lesquels,

- dans chacun des composés du groupe (A1), les radicaux R1, R1' et R1" représentent, indépendamment les uns des autres, un groupe acyle linéaire, saturé ou mono- ou polyinsaturé, $C_8$-$C_{30}$ et
- dans chacun des composés du groupe (A2), l'un des radicaux R2, R2' et R2" représente un atome d'hydrogène et les deux autres radicaux représentent, indépendamment l'un de l'autre, un groupe acyle en $C_8$-$C_{30}$ linéaire, saturé ou mono-ou polyinsaturé, et
- dans chacun des composés du groupe (A3), deux des radicaux R3, R3' et R3" représentent un atome d'hydrogène et le troisième radical représente un groupe acyle en $C_8$-$C_{30}$ linéaire, saturé ou mono- ou polyinsaturé.

**3.** Shampooing selon l'une quelconque des revendications 1 ou 2, **caractérisé en ce que** (B) le mélange de mono- et diesters du mélange d'acides gras (F1) et d'un polyéthylène glycol ayant un poids moléculaire moyen de 200 à 800 g/mol est un mélange des composés des groupes (B1) et (B2),

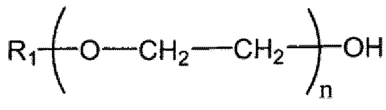
(B1)
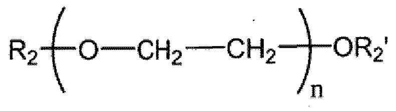
(B2)

dans lesquels

- dans chacun des composés du groupe (B1), R1 est un groupe acyle en $C_8$-$C_{30}$ linéaire, saturé ou mono- ou polyinsaturé, et
- dans chacun des composés du groupe (B2), R2 et R2' représentent indépendamment un groupe acyle linéaire, saturé ou mono- ou polyinsaturé en $C_8$-$C_{30}$, et
- dans chacun des composés des groupes (B1) et (B2), n représente indépendamment un nombre entier de 1 à 50, de préférence de 2 à 40, de manière davantage préférée de 3 à 30 et de manière particulièrement préférée de 4 à 20.

**4.** Shampooing selon l'une quelconque des revendications 1 à 3, **caractérisé en ce que** le mélange d'acides gras (F1) est un mélange d'acides gras dont la composition correspond à celle de l'huile de tournesol.

**5.** Shampooing selon l'une quelconque des revendications 1 à 4, **caractérisé en ce qu'**il contient - par rapport au poids total du shampooing - les mélanges (A) et (B) en une quantité totale [(A) + (B)] de 0,4 à 3,5 % en poids, de préférence de 0,5 à 2,5 % en poids, de manière davantage préférée de 0,6 à 2,0 % en poids, de manière davantage préférée encore de 0,7 à 1,5 % en poids et de manière tout particulièrement préférée de 0,8 à 1,4 % en poids.

**6.** Shampooing selon l'une quelconque des revendications 1 à 5, **caractérisé en ce qu'**il contient les mélanges (A) et (B) connus sous le nom INCI d'ester d'huile de tournesol PEG-8.

**7.** Shampooing selon l'une quelconque des revendications 1 à 6, **caractérisé en ce que**, par rapport au poids total du shampooing, la quantité totale de toutes les huiles végétales contenues dans le shampooing, qui sont différentes des triesters du mélange d'acides gras (F1) et du glycérol, est de 0,20 % en poids au maximum, de préférence de 0,15 % en poids au maximum et de manière davantage préférée de 0,10 % en poids au maximum et de manière tout particulièrement préférée de 0,05 % en poids.

**8.** Shampooing selon l'une quelconque des revendications 1 à 7, **caractérisé en ce que**, par rapport au poids total du shampooing, la quantité totale de tous les composés silicones contenus dans le shampooing est de 0,20 % en poids maximum, de préférence de 0,15 % en poids maximum, de manière davantage préférée de 0,10 % en poids maximum et de manière tout particulièrement préférée de 0,01 % en poids maximum.

**9.** Shampooing selon l'une quelconque des revendications 1 à 8, **caractérisé en ce qu'**il contient en outre (C) de l'huile de ricin hydrogénée PEG-40, le rapport pondéral de [(A)+(B)]/(C) étant de 4,0 à 20,0, de préférence de 6,0 à 16,0, et de manière particulièrement préférée de 8,0 à 14,0.

**10.** Shampooing selon l'une quelconque des revendications 1 à 9, **caractérisé en ce que**, par rapport au poids total du shampooing, la quantité totale de tous les polymères anioniques à base d'acide (méth)acrylique contenus dans le shampooing est de 0,25 % en poids au maximum, de préférence de 0,20 % en poids au maximum, de manière

davantage préférée de 0,10 % en poids au maximum et de manière particulièrement préférée de 0,01 % en poids au maximum.

**11.** Shampooing selon l'une quelconque des revendications 1 à 10, **caractérisé en ce que**, par rapport au poids total du shampooing, la quantité totale de chlorure de sodium contenue dans le shampooing est de 0,25 % en poids au maximum, de préférence de 0,20 % en poids au maximum, de manière davantage préférée de 0,10 % en poids au maximum et de manière particulièrement préférée 0,01 % en poids au maximum.

**12.** Shampooing selon l'une quelconque des revendications 1 à 11 , **caractérisé en ce que**, par rapport au poids total du shampooing, la quantité totale de tous les polysaccharides anioniques et non ioniques contenus dans le shampooing est de 0,25 % en poids au maximum et de préférence de 0,20 % en poids au maximum et de manière davantage préférée de 0,10 % en poids au maximum et de manière particulièrement préférée de 0,01 % en poids au maximum.

**13.** Shampooing selon l'une quelconque des revendications 1 à 12, **caractérisé en ce qu'**il possède une viscosité de 7 000 à 12 000 mPas (20 °C/viscosimètre Brookfield/broche 5/20 tr/min).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9308787 A1